# FASCICULE DE BREVET EUROPEEN

(11) **EP 2 493 909 B1**
(45) Date de publication et mention de la délivrance du brevet: **21.05.2014**
(21) Numéro de dépôt: 10782400.5
(22) Date de dépôt: 28.10.2010
(51) Int. Cl.: C07K 1/02

(54) **PROCEDE DE LIGATION NATIVE DE POLYPEPTIDES**
VERFAHREN ZUR NATIVEN LIGATION VON POLYPEPTIDEN
METHOD FOR NATIVE LIGATION OF POLYPEPTIDES

(30) Priorité: 29.10.2009 FR 0957639
(43) Date de publication de la demande: 05.09.2012
(73) Titulaire: Centre National de la Recherche Scientifique (CNRS), 75794 Paris Cedex 16 (FR); INSTITUT PASTEUR DE LILLE, 59019 Lille Cédex (FR)
(72) Inventeur: MHIDIA, Reda, F-59260 Lille (FR); DHEUR, Julien, F-59480 La Bassee (FR); OLLIVIER, Nathalie, F-59800 Lille (FR); MELNYK, Oleg, F-59112 Annoeullin (FR)
(74) Mandataire: Corizzi, Valérie
(86) Numéro de dépôt international: PCT/IB2010/054897
(87) Numéro de publication internationale: WO 2011/051906

(56) Documents cités:
- WO-A1-98/28434
- AUFORT M ET AL: "Synthesis and biochemical evaluation of a cyclic RGD oxorhenium complex as new ligand of alphaVbeta3 integrin", EUROPEAN JOURNAL OF MEDICINAL CHEMISTRY, EDITIONS SCIENTIFIQUE ELSEVIER, PARIS, FR LNKD- DOI:10.1016/J.EJMECH.2009.02.022, vol. 44, no. 9, 1 septembre 2009 (2009-09-01), pages 3394-3401, XP026322161, ISSN: 0223-5234 [extrait le 2009-02-27]

## Description

### DOMAINE DE L'INVENTION

La présente invention concerne un procédé de ligation native de polypeptides. L'invention concerne également des polypeptides fonctionnalisés utiles pour mettre en oeuvre ce procédé de ligation native, ainsi qu'un procédé de fabrication de ces polypeptides fonctionnalisés. L'invention concerne également un composé amine ainsi qu'une résine fonctionnalisée, utiles pour la mise en oeuvre du procédé de fabrication des polypeptides fonctionnalisés.

### ARRIERE-PLAN TECHNIQUE

La synthèse de polypeptides par des méthodes conventionnelles en phase solide, acide aminé par acide aminé, est limitée par des rendements faibles lorsque les polypeptides synthétisés sont de grande taille. Afin de surmonter cette limitation, il est connu d'assembler deux polypeptides par ligation chimique afin de produire un polypeptide plus long.

De manière générale, on souhaite que le lien entre les polypeptides assemblés par ligation soit natif, c'est-à-dire corresponde à la structure naturelle des polypeptides.

La principale méthode de ligation native existant à ce jour est celle de Kent et Dawson, qui est décrite par exemple dans les documents WO 96/34878 et WO 98/28434. Cette méthode est fondée sur une réaction chimiosélective entre un peptide thioester (en C terminal) et un cystéinyl-peptide. Le principal inconvénient de cette méthode est que la fabrication des peptides thioester nécessite des procédés chimiques complexes.

Une méthode alternative est la ligation dite de Staudinger, décrite dans les documents WO 01/68565 et WO 01/87920. Celle-ci comprend la réaction d'un phosphinothioester avec un azide et l'hydrolyse des réactifs combinés pour former une liaison amide. Cette méthode est difficilement applicable à l'échelle industrielle.

Une troisième méthode, décrite dans le document WO 2007/037812, repose sur la réaction d'un α-cétoacide avec une amine dans une réaction de condensation décarboxylative. Toutefois, les cétoacides sont des molécules qui sont difficiles à fabriquer et à incorporer dans des peptides. Aussi, cette troisième méthode est difficilement applicable dans les laboratoires de synthèse peptidique ne disposant pas des moyens de réaliser des synthèses organiques complexes.

Il existe donc un réel besoin de mettre au point un nouveau procédé de ligation native de polypeptides, qui soit à la fois efficace et plus simple à mettre en oeuvre, y compris à l'échelle industrielle.

### RESUME DE L'INVENTION

L'invention concerne en premier lieu un procédé de fabrication d'un polypeptide de formule :

(III) X₁-X"-X₂

X₁ et X₂ représentant chacun un fragment peptidique, et X" représentant un résidu d'acide aminé comportant une fonction thiol, ledit procédé comprenant au moins une étape de réaction de ligation entre un polypeptide de formule :

(I) X₁-N(CH₂CH₂SH)₂

et un polypeptide de formule :

(II) H-X"-X₂.

Selon un mode de réalisation, la réaction de ligation est effectuée en mettant en contact un polypeptide de formule : 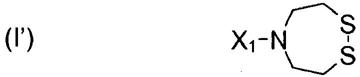
avec le polypeptide de formule (II), en présence d'au moins un composé réducteur des liaisons disulfure.

Selon un mode de réalisation, X₂ représente un fragment peptidique de formule

(IV) X₂'-(Xᵢ"-Xᵢ')_{i=3...n}

n étant un entier supérieur ou égal à 3, chaque Xᵢ", pour i entier compris entre 3 et n, représentant un résidu d'acide aminé comportant une fonction thiol, et chaque Xᵢ', pour i entier compris entre 2 et n, représentant un fragment peptidique ; le procédé comprenant, avant l'étape de réaction de ligation entre le polypeptide de formule (I) et le polypeptide de formule (II), une succession de n-2 étapes de réaction de ligation, la j^{ème} étape de réaction de ligation, pour j entier compris entre 1 et n-2, étant une réaction de ligation entre un polypeptide de formule :

(V) H-Xₙ₋ₜ"Xₙ₋ⱼ'- N(CH₂CH₂SH)₂

dans lequel la fonction amine et / ou la fonction thiol du résidu Xₙ₋ⱼ" est protégée, et un polypeptide de formule :

(VI) H-(Xᵢ"-Xᵢ')_{i=(n-j+1)...n}

pour former un polypeptide de formule :

(VII) H-(Xᵢ"-Xᵢ')_{i=(n-j)...n}

le polypeptide de formule (VII) subissant une déprotection de la fonction thiol du résidu Xₙ₋ⱼ" à l'issue de la réaction de ligation.

Selon un mode de réalisation, une ou plusieurs des n-2 étapes de réaction de ligation entre le polypeptide de formule (V) et le polypeptide de formule (VI) est effectuée en mettant en contact un polypeptide de formule : 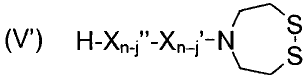 avec le polypeptide de formule :

(VI) H-(Xᵢ"-Xᵢ')_{i=(n-j+1)...n}

j étant un entier compris entre 1 et n-2, en présence d'au moins un composé réducteur des liaisons disulfure.

L'invention concerne également un procédé de fabrication d'un polypeptide cyclique de formule : 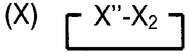
X₂ représentant un fragment peptidique, et X" représentant un résidu d'acide aminé comportant une fonction thiol, ledit procédé comprenant au moins une étape de réaction de ligation d'un polypeptide de formule :

(XI) H-X"-X₂-N(CH₂CH₂SH)₂

avec lui-même.

Selon un mode de réalisation, la réaction de ligation est effectuée en mettant en contact un polypeptide de formule : 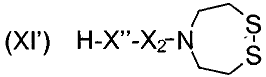 avec au moins un composé réducteur des liaisons disulfure.

Selon un mode de réalisation de l'un quelconque des procédés précédents, la ou les réactions de ligation sont effectuées en milieu aqueux, de préférence à un pH compris entre 6,5 et 8,5, de manière plus particulièrement préférée entre 7 et 8, et idéalement d'environ 7,5.

Selon un mode de réalisation de l'un quelconque des procédés précédents, la ou les réactions de ligation sont effectuées en présence d'au moins un composé réducteur des liaisons disulfure, de préférence choisi parmi la tris(2-carboxyéthyl)phosphine, l'acide 4-mercaptophénylacétique, le dithiothréitol, le benzylmercaptan et les mélanges de ceux-ci.

L'invention concerne par ailleurs un polypeptide de formule :

(I) X₁-N(CH₂CH₂SH)₂

ou de formule : 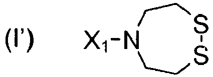 dans lesquelles X₁ représente un fragment peptidique et le groupement -N(CH₂ CH₂ SH)₂ ou 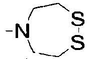 est lié à la terminaison C=O du résidu d'acide aminé du fragment peptidique X₁ qui est en position C-terminale.

Selon un mode de réalisation, X₁ comprend entre 2 et 300 résidus d'acides aminés, de préférence entre 5 et 100 résidus d'acides aminés, de manière plus particulièrement préférée entre 8 et 50 résidus d'acides aminés.

L'invention concerne également un procédé de fabrication d'un polypeptide de formule :

(I) X₁-N(CH₂CH₂SH)₂

X₁ représentant un fragment peptidique, comprenant au moins une étape de synthèse peptidique et une étape de fonctionnalisation C-terminale.

Selon un mode de réalisation, l'étape de synthèse peptidique précède l'étape de fonctionnalisation ; l'étape de synthèse peptidique fournit un polypeptide de formule :

(IX) X₁-OH

comportant de préférence des groupements protecteurs sur ses fonctions amines et carboxyliques, à l'exception de sa fonction carboxylique C-terminale ; et l'étape de fonctionnalisation comprend :
- la réaction du polypeptide de formule (IX) avec le composé amine de formule :

   (VIII) NH(CH₂-CH₂-S-G₁)₂

   dans laquelle G₁ représente un groupement protecteur, ledit groupement protecteur formant de préférence une fonction thioéther, thioester ou disulfure, et étant de manière plus particulièrement préférée le groupement triphénylméthyle, en phase liquide, pour former le polypeptide de formule (I) ;
- éventuellement la déprotection du polypeptide de formule (I).

L'invention a également pour objet un support de résine polymère pour la synthèse de polypeptides en phase solide, comprenant un squelette principal et des groupements fonctionnels NH-(CH₂CH₂-S-Trt-)₂ ou des groupements fonctionnels NH-(CH₂CH₂-S-Trt-CO-NH-)₂, où Trt représente un groupement triphénylméthyle éventuellement substitué par un ou plusieurs substituants, notamment choisis parmi les substituants chlore, méthoxy, méthyle, fluor et cyano ; dans lequel les groupements fonctionnels NH-(CH₂CH₂-S-Trt-)₂ sont liés au squelette principal par les deux groupements triphénylméthyle, ou les groupements fonctionnels NH-(CH₂CH₂-S-Trt-CO-NH-)₂ sont liés au squelette principal par les deux groupements amines.

L'invention a également pour objet un support de résine polymère pour la synthèse de polypeptides en phase solide, comprenant un squelette principal et des groupements fonctionnels G₂-AA-N-(CH₂CH₂-S-Trt-)₂ ou des groupements fonctionnels G₂-AA-N-(CH₂CH₂-S-Trt-CO-NH-)₂, où Trt représente un groupement triphénylméthyle éventuellement substitué par un ou plusieurs substituants, notamment choisis parmi les substituants chlore, méthoxy, méthyle, fluor et cyano ; AA représente un résidu d'acide aminé comportant éventuellement un ou plusieurs groupements protecteurs ; G₂ représente un atome d'hydrogène ou un groupement protecteur de fonction amine ; dans lequel les groupements fonctionnels G₂-AA-N(CH₂CH₂-S-Trt-)₂ sont liés au squelette principal par les deux groupements triphénylméthyle ou les groupements fonctionnels G₂-AA-N-(CH₂CH₂-S-Trt-CO-NH-)₂ sont liés au squelette principal par les deux groupements amines.

Selon un mode de réalisation des supports de résine polymère précédents, le squelette principal est choisi parmi les squelettes polystyrène, polyacrylamide, polyéthylèneglycol, cellulose, polyéthylène, polyester, latex, polyamide, polydiméthylacrylamide, copolymère polyéthylèneglycol-polystyrène, copolymère polyéthylèneglycol-polyacrylamide et leurs dérivés.

L'invention concerne également un procédé de fabrication d'un support de résine polymère pour la synthèse de polypeptides en phase solide, comprenant :
- la fourniture d'une résine polymère ;
- la fonctionnalisation de la résine polymère par réaction avec le composé amine de formule :

   (VIII') NH(CH₂-CH₂-S-H)₂

Selon un mode de réalisation du procédé de fabrication de support de résine polymère, le procédé comprend, préalablement à l'étape de fonctionnalisation de la résine polymère :
- la fourniture d'un composé amine de formule :

   (VIII) NH(CH₂-CH₂-S-G₁)₂

   dans laquelle G₁ représente un groupement protecteur, ledit groupement protecteur formant de préférence une fonction thioéther, thioester ou disulfure, et étant de manière plus particulièrement préférée le groupement triphénylméthyle ;
- la déprotection de ce composé amine pour obtenir le composé amine de formule (VIII').

Selon un mode de réalisation du procédé de fabrication du polypeptide de formule (I) :
- l'étape de fonctionnalisation précède l'étape de synthèse peptidique ;
- l'étape de fonctionnalisation comprend :
   ■ le couplage d'un acide aminé à un support de résine polymère comprenant un squelette principal et des groupements fonctionnels NH-(CH₂CH₂-S-Trt-)₂ ou des groupements fonctionnels NH-(CH₂CH₂-S-Trt-CO-NH-)₂, tel que décrit ci-dessus, pour fournir un support amorce ; ou
   ■ la fourniture d'un support amorce qui est un support de résine polymère comprenant un squelette principal et des groupements fonctionnels G₂-AA-N-(CH₂CH₂-S-Trt-)₂ ou des groupements fonctionnels G₂-AA-N-(CH₂CH₂-S-Trt-CO-NH-)₂, tel que décrit ci-dessus;
- l'étape de synthèse peptidique comprend une succession de couplages d'acides aminés sur le support amorce.

Selon un mode de réalisation de ce procédé, le couplage d'un acide aminé au support de résine polymère comprend la mise en présence du support de résine polymère avec un halogénure d'acide aminé ou avec un acide aminé et un agent d'activation, choisi de préférence parmi le PyBOP, le BOP, le PyBROP, de manière plus particulièrement préférée le PyBROP.

L'invention a aussi pour objet un procédé de fabrication d'un polypeptide de formule : 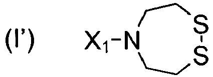
dans laquelle X₁ représente un fragment peptidique, comprenant une étape d'oxydation d'un polypeptide de formule :

(I) X₁-N(CH₂CH₂SH)₂

de préférence au contact de l'air, ou en présence de I₂ ou de diamide, et dans un tampon, ladite étape d'oxydation étant de préférence précédée d'une étape de fabrication du polypeptide de formule (I) selon le procédé décrit ci-dessus.

Selon un mode de réalisation du procédé de fabrication d'un polypeptide de formule (III) ou (X), celui-ci comprend une étape de fabrication du polypeptide de formule (I) et / ou (V) ou (XI) qui est selon le procédé de fabrication du polypeptide de formule (I) décrit ci-dessus, ou une étape de fabrication du polypeptide de formule (I') et / ou (V') ou (XI') selon le procédé de fabrication du polypeptide de formule (I') décrit ci-dessus.

L'invention concerne également un procédé de fabrication d'une composition pharmaceutique comprenant :
- la fabrication d'un polypeptide selon le procédé de fabrication d'un polypeptide de formule (III) ou (X) décrit ci-dessus, et
- la formulation de ce polypeptide avec un ou plusieurs adjuvants pharmaceutiquement acceptables.

L'invention concerne également un procédé de fabrication d'un dispositif de diagnostic comprenant :
- la fabrication d'un polypeptide selon le procédé de fabrication d'un polypeptide de formule (III) ou (X) décrit ci-dessus, et
- la formulation de ce polypeptide sous une forme adaptée à une utilisation diagnostique.

L'invention a encore pour objet un composé amine de formule :

(VIII") NH(CH₂-CH₂-S-Trt)₂

où Trt représente le groupement triphénylméthyle.

La présente invention permet de surmonter les inconvénients de l'état de la technique. Elle fournit plus particulièrement un procédé de ligation native de polypeptides, qui est à la fois efficace et plus simple à mettre en oeuvre que les procédés antérieurs, y compris à l'échelle industrielle.

Cela est accompli grâce à la mise au point d'un schéma réactionnel dans lequel un polypeptide modifié à l'extrémité C-terminale par un groupement bis(mercaptoéthyl)amino réagit avec un polypeptide présentant une cystéine à l'extrémité N-terminale (ou un autre acide aminé comportant une fonction thiol) pour former une liaison amide native.

Selon certains modes de réalisation particuliers, l'invention présente également une ou de préférence plusieurs des caractéristiques avantageuses énumérées ci-dessous.
- La méthode de ligation selon l'invention utilise éventuellement des réactifs polypeptidiques non protégés, en particulier lorsque l'on effectue une seule ligation. L'utilisation de polypeptides protégés est délicate du fait de leur faible solubilité, et parce qu'elle exige en outre une étape de déprotection après la ligation, entraînant un coût supplémentaire et une possibilité de dégradation. A l'inverse, l'invention permet donc d'éviter les inconvénients liés aux polypeptides protégés, en particulier lorsque l'on effectue une seule ligation. Le procédé selon l'invention conduit directement à la formation d'un lien natif au point de ligation, sans qu'il soit nécessaire d'effectuer une déprotection après la ligation.
- La méthode de ligation selon l'invention repose sur l'utilisation de polypeptides modifiés par des groupements fonctionnels stables chimiquement, et qui sont faciles à introduire en utilisant les techniques de synthèse peptidique conventionnelles.
- L'invention permet d'utiliser des acides aminés protéinogéniques pour la synthèse des fragments peptidiques. Ainsi, il est inutile de recourir à la fabrication de dérivés d'acides aminés (tels que des cétoacides par exemple), qui alourdit considérablement la synthèse.
- L'assemblage des réactifs polypeptidiques peut être réalisé de façon classique, par exemple en utilisant la chimie de Fmoc/tert-butyle. Par conséquent, le procédé selon l'invention est compatible avec les processus de synthèse industriels et automatisés actuellement disponibles. Les acides aminés et les supports solides appropriés sont actuellement disponibles en grande quantité et à bas coût.
- L'invention prévoit une ligation en milieu aqueux, qui est donc compatible avec la solubilité des peptides et des protéines.
- La réaction de ligation selon l'invention peut être effectuée efficacement à un pH proche de 7,5, c'est-à-dire en conditions compatibles avec les polypeptides complexes ou protéines.
- La réaction de ligation selon l'invention offre une possibilité d'auto-ligation d'un polypeptide, et donc de fabrication de polypeptides cycliques.

### BREVE DESCRIPTION DES FIGURES

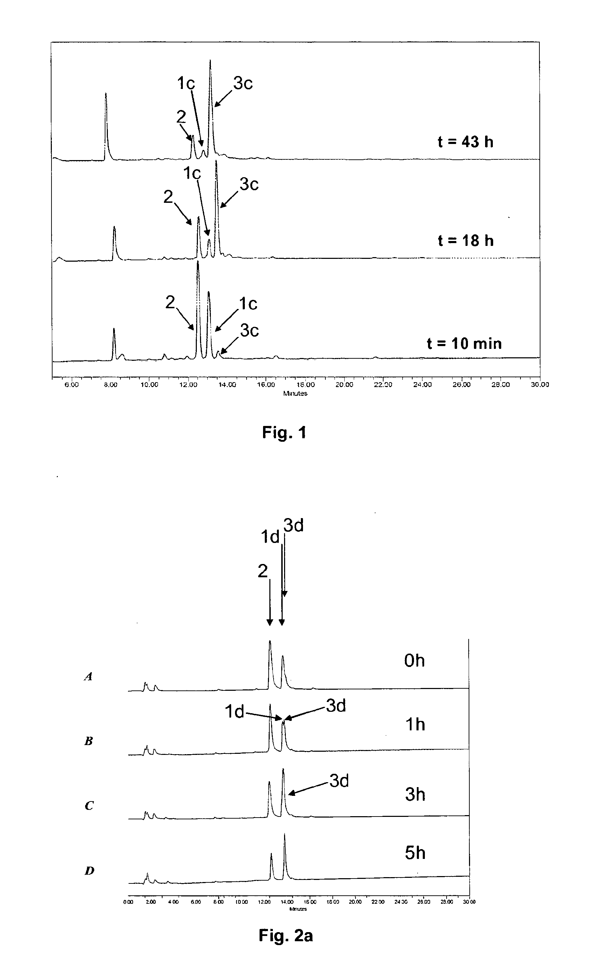

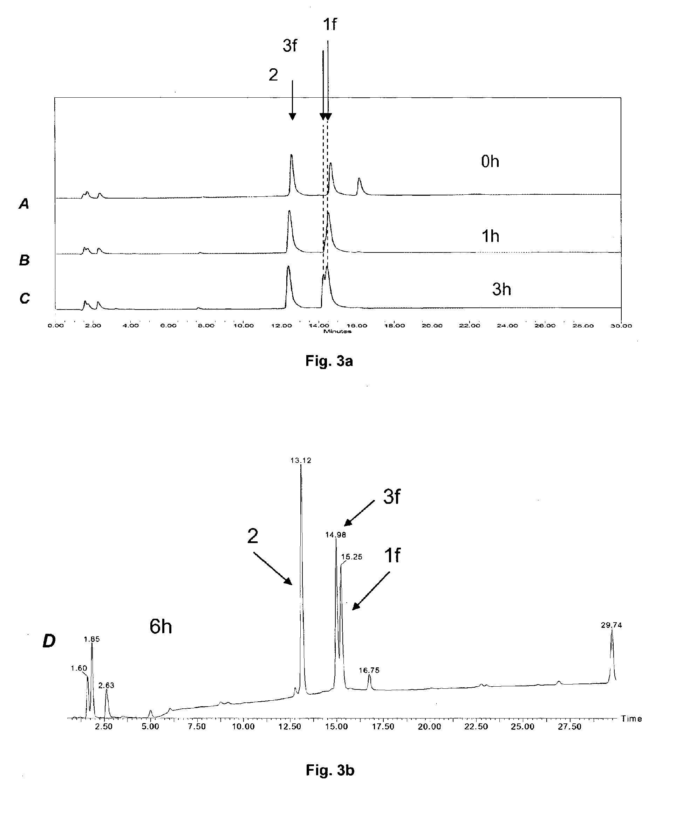

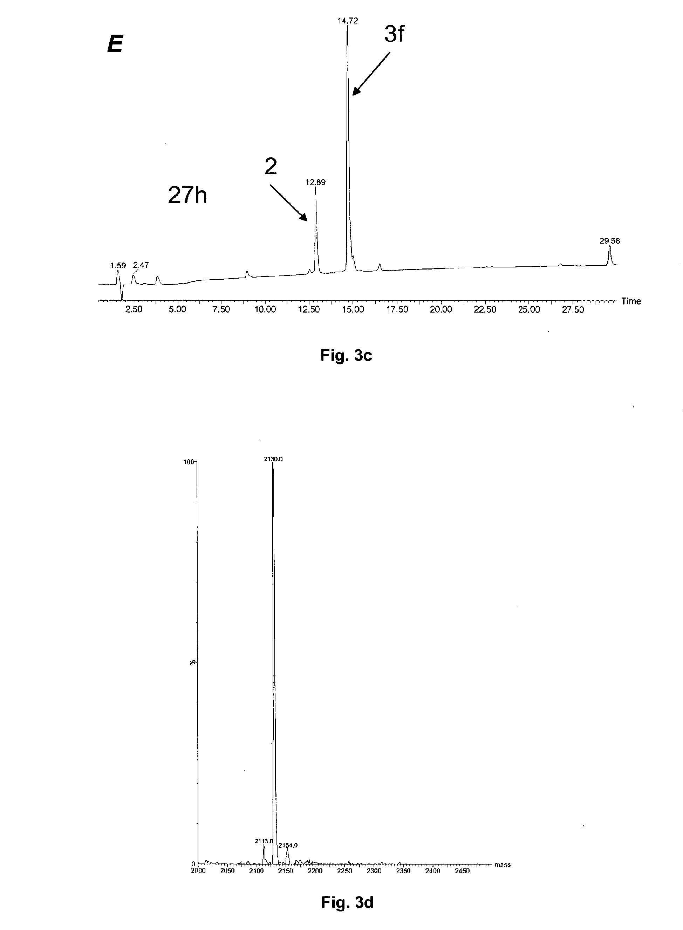

La **Fig. 1** représente le suivi en RP-HPLC (chromatographie liquide haute performance en phase inverse) de la ligation native entre le polypeptide 1c et le polypeptide 2, pour obtenir le polypeptide 3c, selon l'exemple 7. Le tracé inférieur correspond à l'instant t=10 min ; le tracé du milieu correspond à l'instant t=18 h ; et le tracé supérieur correspond à l'instant t=43 h.

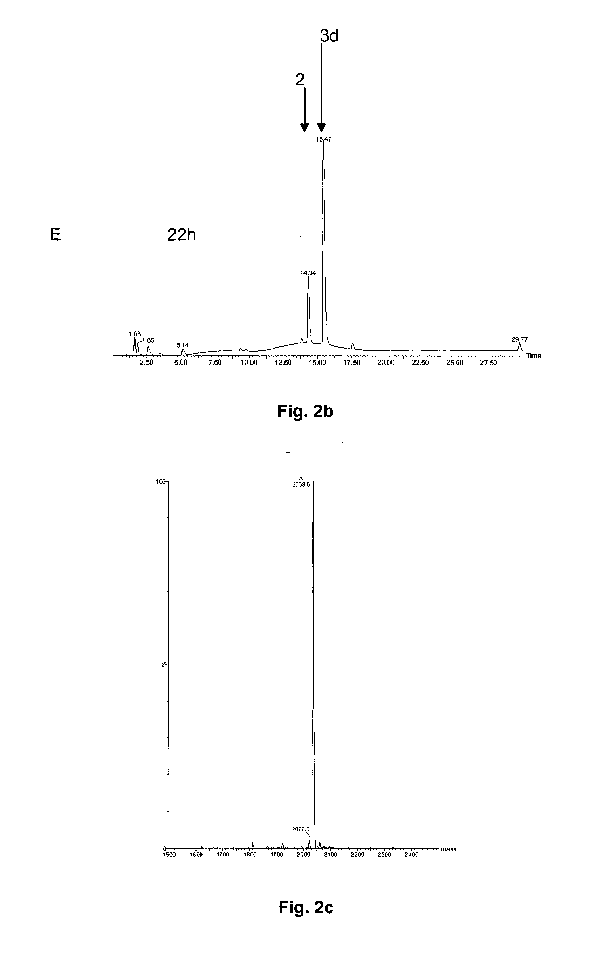

Les **Fig. 2a** et **2b** représentent le suivi en RP-HPLC (chromatographie liquide haute performance en phase inverse) de la ligation native entre le polypeptide 1d et le polypeptide 2, pour obtenir le polypeptide 3d, selon l'exemple 7. Le tracé A correspond à l'instant t=0 ; le tracé B correspond à l'instant t=1 h ; le tracé C correspond à l'instant t=3 h ; le tracé D correspond à l'instant t=5 h ; et le tracé E correspond à l'instant t=22 h.
La **Fig**. **2c** représente le spectre de spectrométrie de masse déconvolué pour le pic du polypeptide 3d obtenu à l'instant t=22h (Fig. 2b).
Les **Fig**. **3a** à **3c** représentent le suivi en RP-HPLC (chromatographie liquide haute performance en phase inverse) de la ligation native entre le polypeptide 1f et le polypeptide 2, pour obtenir le polypeptide 3f, selon l'exemple 7. Le tracé A correspond à l'instant t=0 ; le tracé B correspond à l'instant t=1 h ; le tracé C correspond à l'instant t=3 h ; le tracé D correspond à l'instant t=6 h ; et le tracé E correspond à l'instant t=27 h.
La **Fig**. **3d** représente le spectre de spectrométrie de masse déconvolué pour le pic du polypeptide 3f obtenu à l'instant t=27h (Fig. 3c).

### DESCRIPTION DE MODES DE REALISATION DE L'INVENTION

L'invention est maintenant décrite plus en détail et de façon non limitative dans la description qui suit.

Par « polypeptide » on entend, dans le cadre de la présente demande, une chaîne linéaire de résidus d'acides aminés (en nombre supérieur ou égal à deux) reliés par des liaisons peptidiques. Les « polypeptides » au sens de la présente demande peuvent donc par exemple être des oligopeptides, peptides ou protéines selon l'acception conventionnelle de ces termes. Les résidus d'acides aminés présents dans les polypeptides selon l'invention peuvent être choisis parmi les résidus d'acides aminés protéinogéniques ou non. De préférence, ils sont choisis parmi les vingt résidus d'acides aminés protéinogéniques.

La notation des polypeptides s'effectue de l'extrémité N-terminale vers l'extrémité C-terminale. Les résidus d'acides aminés présents le long de la chaîne polypeptidique sont notés selon le code usuel à une lettre ou à trois lettres. Un résidu d'acide aminé est un fragment de polypeptide de formule -NH-(CH-R)-(C=O)-, dans laquelle R représente une chaîne latérale, différente d'un acide aminé à l'autre.

Par « fragment peptidique » on entend, dans le cadre de la présente demande, une portion de polypeptide comprenant au moins un résidu d'acide aminé. Un fragment peptidique, au sens de la présente demande, peut donc être par exemple : une séquence de résidus d'acides aminés (telle que -AHG- ou -Ala-His-Gly-) si le fragment peptidique ne comprend ni l'extrémité N-terminale ni l'extrémité C-terminale du polypeptide ; ou bien une séquence de résidus d'acides aminés présentant un groupement à son extrémité N-terminale (telle que H-AHG- ou H-Ala-His-Gly-) si le fragment peptidique comprend l'extrémité N-terminale du polypeptide ; ou bien une séquence de résidus d'acides aminés présentant un groupement à son extrémité C-terminale (telle que -AHG-OH ou -Ala-His-Gly-OH) si le fragment peptidique comprend l'extrémité C-terminale du polypeptide.

### Ligation native de polypeptides

L'invention fournit une méthode de ligation native de polypeptides, selon laquelle un polypeptide de formule :

(I) X₁-N(CH₂CH₂SH)₂

réagit avec un polypeptide de formule :

(II) H-X"-X₂

pour fournir un polypeptide de formule :

(III) X₁-X"-X₂.

Le polypeptide de formule (I) comprend un fragment peptidique X₁ (ledit fragment peptidique comprenant l'extrémité N-terminale du polypeptide) et un groupement fonctionnel -N(CH₂CH₂SH)₂ à l'extrémité C-terminale (lié à la terminaison (C=O) du résidu d'acide aminé en position C-terminale).

Le fragment peptidique X₁ est de la forme Y₁-AA₁AA₂...AAₙ. Y₁ est un groupement N-terminal, de préférence un atome d'hydrogène, mais éventuellement aussi tout groupement substituant pour les amines primaires ou secondaires connu de l'homme du métier, par exemple un groupement acyle et notamment un groupement acétyle. n est un nombre entier supérieur ou égal à 2. Chaque AA₁ représente un résidu d'acide aminé.

Un exemple de polypeptide de formule (I) est le polypeptide 1a (voir exemple 3 ci-dessous) de formule :

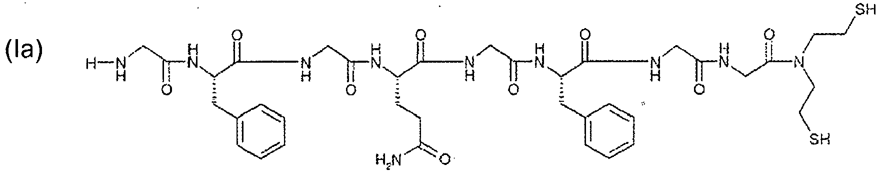

Dans cet exemple, le fragment peptidique X₁ est H-GFGQGFGG.

Le polypeptide de formule (I) comprend de préférence entre 2 et 300 résidus d'acides aminés, de préférence entre 5 et 100 résidus d'acides aminés, de manière plus particulièrement préférée entre 8 et 50 résidus d'acides aminés.

Le polypeptide de formule (II) comprend un atome d'hydrogène et un résidu X" à l'extrémité N-terminale. Le résidu X" est un résidu d'acide aminé comportant une fonction thiol. Cette fonction thiol peut être en particulier une fonction beta-amino thiol (auquel cas le résidu X" représente de préférence le résidu cystéine) ou une fonction gamma-amino thiol (auquel cas le résidu X" représente de préférence le résidu homocystéine).

Dans toute la description qui suit, selon un mode de réalisation particulier, X" peut se lire comme représentant un résidu cystéine (Cys).

Selon la notation utilisée ci-dessus, X₂ représente un fragment peptidique, qui comprend l'extrémité C-terminale du polypeptide de formule (II) ainsi que l'ensemble des résidus d'acides aminés de ce polypeptide, à l'exception du résidu N-terminal.

Le fragment peptidique X₂ est de la forme AA₂'AA₃'...AAₙ'-Y₂. Y₂ est un groupement terminal, de préférence un groupement -OH ou -NH₂ ou encore un groupement -OR ou -NRR', R et R' représentant chacun un groupement alkyle ou aryle. n est un nombre entier supérieur ou égal à 2. Chaque AAᵢ' représente un résidu d'acide aminé.

Le polypeptide de formule (II) comprend de préférence entre 2 et 300 résidus d'acides aminés, de préférence entre 5 et 100 résidus d'acides aminés, de manière plus particulièrement préférée entre 8 et 50 résidus d'acides aminés.

Le polypeptide de formule (II) peut être par exemple obtenu par une méthode de synthèse peptidique usuelle, notamment une méthode de synthèse en phase solide. Il peut également être obtenu au moyen d'une ligation native précédente (voir ci-dessous).

Chacun des polypeptides de formule (I) et (II) comprend de préférence uniquement des résidus d'acides aminés choisis parmi les vingt résidus d'acides aminés protéinogéniques. Toutefois, selon un mode de réalisation particulier, les polypeptides de formule (I) et (II) comprennent un ou plusieurs résidus d'acides aminés non-protéinogéniques.

Les résidus d'acides aminés des polypeptides de formule (I) et (II) peuvent éventuellement être protégés par des groupements protecteurs des chaînes latérales.

Pour que la réaction de ligation s'effectue correctement, la présence des deux groupes thiol libres sur le polypeptide de formule (I) est essentielle. La ligation est dite native car le fragment peptidique X₁ est relié au fragment peptidique X"-X₂ par une liaison amide.

Il est possible d'effectuer la réaction de ligation ci-dessus en mettant en présence le polypeptide de formule (II) avec un polypeptide de formule : 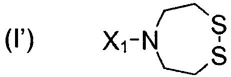 à condition d'utiliser lors de la réaction un composé réducteur des liaisons disulfure, qui peut être de préférence un composé thiol tel que l'acide 4-mercaptophénylacétique (MPAA), le dithiothréitol (DTT), le thiophénol (et ses dérivés), un alkylthiol (notamment le benzylmercaptan) ou encore une phosphine telle que la tris(2-carboxyéthyl)phosphine (TCEP). L'utilisation conjointe de plusieurs de ces composés est également appropriée, par exemple l'utilisation de MPAA et de TCEP.

En effet, le polypeptide de formule (I') est alors réduit *in situ* et fournit le polypeptide de formule (I) pour la réaction de ligation.

De manière générale, la réaction de ligation à partir du polypeptide de formule (I') en tant que réactif peut être plus pratique à mettre en oeuvre que la réaction de ligation directement avec le polypeptide de formule (I). En effet, le polypeptide de formule (I) a naturellement tendance à s'oxyder en le polypeptide de formule (I'), notamment sous l'action de l'oxygène de l'air. Par exemple, il est possible que le polypeptide de forme ouverte (I) s'oxyde dans le temps lorsqu'il est stocké sous forme lyophilisée. En d'autres termes, une préparation du polypeptide de formule (I) contient généralement nécessairement en partie le polypeptide de formule (I'). La présence de ces deux formes peut compliquer la caractérisation et la purification. C'est pourquoi il peut être plus simple de mettre en oeuvre la réaction de ligation par mise en contact du polypeptide de formule (I') avec le polypeptide de formule (II), le polypeptide à terminaison cyclique de formule (I') étant réduit *in situ* en le polypeptide ouvert de formule (I).

Pour les mêmes raisons, même lorsque la réaction de ligation est effectuée directement par mise en contact du polypeptide de formule (I) avec le polypeptide de formule (II), il est préférable d'utiliser lors de la réaction un ou plusieurs des composés réducteurs des liaisons disulfure mentionnés ci-dessus.

De préférence, le MPAA, lorsqu'il est présent, est utilisé à une concentration comprise entre 1 et 500 mM, par exemple à une concentration d'environ 200 mM lors de la réaction.

De préférence, la TCEP lorsqu'elle est présente, est utilisée à une concentration comprise entre 1 et 200 mM, par exemple à une concentration d'environ 80 mM lors de la réaction.

Dans l'hypothèse où le résidu d'acide aminé N-terminal du polypeptide (I) comporte une fonction thiol, celle-ci doit être protégée lors de la ligation, sans quoi on aboutit à une réaction concurrente de cyclisation du polypeptide de formule (I). Alternativement, on peut protéger la fonction amine alpha afin d'éviter la réaction de cyclisation. On peut utiliser par exemple une protection de type thiazolidine, qui protège simultanément le thiol et l'amine alpha.

La réaction de ligation intervient de préférence en phase liquide, et notamment en milieu aqueux, par exemple dans un tampon phosphate. De préférence, cette réaction est effectuée à un pH compris entre 6,5 et 8,5, de manière plus particulièrement préférée à un pH compris entre 7 et 8 et idéalement à un pH voisin de 7,5.

La réaction de ligation est effectuée de préférence à une température comprise entre 0 et 50°C, et idéalement à une température d'environ 37°C. La durée de la réaction est ajustée en fonction du choix des réactifs et des autres conditions de la réaction. La durée appropriée peut également être ajustée selon les résultats d'une analyse de chromatographie liquide - spectrométrie de masse au cours de la réaction. La durée appropriée sera typiquement de quelques heures à quelques jours.

Chacun des polypeptides de formule (I) et (II) est de préférence présent à une concentration comprise entre 0,01 et 50 mM, lors de la réaction. Le rapport de concentration molaire entre les polypeptides de formule (I) et (II) lors de la réaction est de préférence compris entre 2:3 et 3:2.

La réaction de ligation décrite ci-dessus peut être suivie d'une étape de purification du polypeptide de formule (III) obtenu, par exemple par chromatographie en phase liquide ou par toute autre technique usuelle.

### Production d'un polypeptide avec plusieurs ligations natives successives

L'invention permet également de produire des polypeptides en enchaînant successivement plusieurs réactions de ligation telles que décrites ci-dessus. Ceci peut s'avérer approprié pour obtenir des polypeptides de grande taille, par exemple des polypeptides comprenant plus d'environ 100 résidus d'acides aminés. En effet, dans de tels cas la fabrication de polypeptides de formules (I) et (II) par synthèse directe peut avoir un faible rendement, et il est donc avantageux d'utiliser deux ou plus de deux ligations successives, afin d'avoir à synthétiser directement uniquement des polypeptides comprenant par exemple moins d'environ 50 résidus d'acides aminés.

A titre d'exemple, l'utilisation de deux ligations successives permet d'obtenir un polypeptide comprenant environ 150 résidus d'acides aminés sans avoir à synthétiser directement de polypeptide comprenant plus d'environ 50 résidus d'acides aminés ; l'utilisation de trois ligations successives permet d'obtenir un polypeptide comprenant environ 200 résidus d'acides aminés sans avoir à synthétiser directement de polypeptide comprenant plus d'environ 50 résidus d'acides aminés.

Ainsi, le procédé selon l'invention permet d'obtenir un polypeptide de formule (III) dans lequel le fragment peptidique X₂ est de la forme X₂'-X₃"-X₃'- ...-Xₙ"-Xₙ' (n étant un entier supérieur ou égal à 3 ; chaque Xᵢ', pour i entier entre 2 et n, étant un fragment peptidique ; et chaque Xᵢ", pour i entier entre 3 et n, étant un résidu X", c'est-à-dire un résidu d'acide aminé comportant une fonction thiol, et notamment un résidu cystéine selon un mode de réalisation particulier) en utilisant n-1 ligations successives. En d'autres termes le polypeptide obtenu présente dans ce cas la formule :

(III') X₁-X"-X₂'-X₃"-X₃'-...-Xₙ"-Xₙ'

La première réaction de ligation fait intervenir d'une part un polypeptide de formule

(Va) H-Xₙ₋₁"-Xₙ₋₁'-N(CH₂CH₂SH)₂

et d'autre part un polypeptide de formule

(Vla) H-Xₙ"-Xₙ'.

Cette réaction de ligation est exactement similaire à celle décrite dans la section précédente. Elle conduit à l'obtention du polypeptide de formule

(VIb) H-Xₙ₋₁"-Xₙ₋₁'-Xₙ"-Xₙ'.

La fonction thiol ou la fonction amine N-terminale (ou la fonction thiol et la fonction amine) du résidu d'acide aminé Xₙ₋₁" doit être protégée dans le polypeptide de formule (Va) lors de la ligation, sans quoi on aboutit à une réaction concurrente de cyclisation du polypeptide de formule (Va). On peut utiliser pour ce faire par exemple une protection de type thiazolidine.

A l'issue de la réaction de ligation, la fonction thiol du résidu d'acide aminé Xₙ₋₁" doit être déprotégée dans le polypeptide de formule (Vlb) afin de permettre la réaction de ligation suivante.

La deuxième réaction de ligation fait intervenir d'une part un polypeptide de formule :

(Vb) H-Xₙ₋₂"-Xₙ₋₂'-N(CH₂CH₂SH)₂

et d'autre part le polypeptide de formule (Vlb) précédemment obtenu.

La fonction thiol du résidu d'acide aminé Xₙ₋₂" doit être protégée dans le polypeptide de formule (Vb), sans quoi on aboutit à une réaction concurrente de cyclisation du polypeptide de formule (Vb). On peut utiliser pour ce faire par exemple une protection de type thiazolidine. La réaction de ligation du polypeptide de formule (Vb) avec le polypeptide de formule (Vlb) est alors suivie d'une déprotection de la fonction thiol du résidu d'acide aminé Xₙ₋₂" préalablement à la réaction de ligation suivante.

Les réactions de ligation suivantes sont du même type. De manière générique, la réaction de ligation numéro j (pour j entier compris entre 1 et n-2) fait intervenir un polypeptide de formule

(V) H-Xₙ₋ⱼ"-Xₙ₋ⱼ'- N(CH₂CH₂SH)₂

et un polypeptide de formule :

(VI) H-(Xᵢ"-Xᵢ')_{i=(n-j+1)...n}

pour former un polypeptide de formule :

(VII) H-(Xᵢ"-Xᵢ')_{i=(n-j)...n}

Enfin, la dernière (c'est-à-dire la n-1^{ème}) réaction de ligation fait intervenir le polypeptide de formule :

(I) X₁-N(CH₂CH₂SH)₂

et le polypeptide de formule :

(II) H-X"-X₂,

qui représente ici H-X"-X₂'-X₃"-X₃'-...-Xₙ"-Xₙ',
pour former le polypeptide de formule :

(III) X₁-X"-X₂, c'est-à-dire

(III') X₁-X"-X₂'-X₃"-X₃'-...-Xₙ"-Xₙ',

Chacune des réactions de ligation successives peut être effectuée comme décrit dans la section « ligation native de polypeptides ». En particulier, il peut être avantageux d'effectuer la réaction de ligation numéro j (pour j entier compris entre 1 et n-2) par mise en contact du polypeptide de formule (VI) avec le polypeptide de formule : 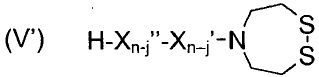 en présence d'un ou plusieurs composés réducteurs de liaisons disulfure mentionnés ci-dessus, le polypeptide de formule (V') étant alors réduit *in situ* et fournissant le polypeptide de formule (V) pour la réaction de ligation.

### Production d'un polypeptide cyclique par auto-ligation native

Les principes utilisés pour la ligation native décrits ci-dessus peuvent également être utilisés pour produire des polypeptides cycliques, par auto-ligation native d'un polypeptide (ligation d'une extrémité du polypeptide avec l'autre extrémité du même polypeptide). De manière générale, l'invention propose ainsi un procédé de fabrication d'un polypeptide cyclique de formule : 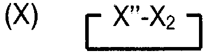
où X₂ représente un fragment peptidique, et X" représente un résidu d'acide aminé comportant une fonction thiol, par une réaction de ligation d'un polypeptide de formule :

(XI) H-X"-X₂-N(CH₂CH₂SH)₂

avec lui-même.

Le polypeptide de formule (XI) comprend de préférence entre 2 et 300 résidus d'acides aminés, de préférence entre 5 et 100 résidus d'acides aminés, de manière plus particulièrement préférée entre 8 et 50 résidus d'acides aminés.

Le polypeptide de formule (XI) comprend un atome d'hydrogène et un résidu X" à l'extrémité N-terminale, X" ayant la signification indiquée ci-dessus. X₂ représente ici un fragment peptidique de la forme AA₁AA₂...AAₙ où n est un nombre entier supérieur ou égal à 1 et chaque AAᵢ représente un résidu d'acide aminé.

Le polypeptide de formule (XI) comprend de préférence uniquement des résidus d'acides aminés choisis parmi les vingt résidus d'acides aminés protéinogéniques. Toutefois, selon un mode de réalisation particulier, le polypeptide de formule (XI) comprend un ou plusieurs résidus d'acides aminés non- protéinogéniques.

Les résidus d'acides aminés du polypeptide de formule (XI) peuvent éventuellement être protégés par des groupements protecteurs des chaînes latérales.

Il est possible d'effectuer la réaction de ligation ci-dessus en mettant en présence le polypeptide de formule (XI') : 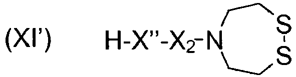
avec au moins un composé réducteur des liaisons disulfure, qui est de préférence tel que décrit ci-dessus. En effet, le polypeptide de formule (XI') est alors réduit *in situ* et fournit le polypeptide de formule (XI) pour la réaction de ligation.

De manière générale, même lorsque la réaction de ligation est effectuée directement à partir du polypeptide de formule (XI), il est préférable d'utiliser lors de la réaction un ou plusieurs des composés réducteurs des liaisons disulfure mentionnés ci-dessus.

En général, la cyclisation du polypeptide de formule (XI) n'est pas gênée par des multimérisations concurrentes, si la réaction est effectuée en conditions suffisamment diluées. On peut par exemple utiliser une concentration de polypeptide de formule (XI) comprise entre 0,01 et 50 mM, typiquement d'environ 1 mM (ou éventuellement entre 0,01 et 0,1 mM en cas de risques sérieux de multimérisations).

Par ailleurs, les conditions préférées de mise en oeuvre de la réaction de ligation sont les mêmes que celles décrites ci-dessus pour la réaction à partir des polypeptides de formule (I) et (II).

La réaction de ligation décrite ci-dessus peut être suivie d'une étape de purification du polypeptide cyclique de formule (X) obtenu, par exemple par chromatographie en phase liquide ou par toute autre technique usuelle.

### Procédé de fabrication des polypeptides de formule (I), (V) et (XI)

Les polypeptides de formule (I), (V) et (XI) sont des composés utiles à la mise en oeuvre de la réaction de ligation décrite ci-dessus. L'invention a donc également pour objet ces polypeptides de formule (I) (ou de formule (V) ou (XI)) en tant que tels, ainsi qu'un procédé permettant de les fabriquer.

Le procédé de fabrication des polypeptides de formule (I) (ou de formule (V) ou (XI)) fait intervenir deux étapes principales :
- une étape de synthèse peptidique ; et
- une étape de fonctionnalisation C-terminale.

L'invention fournit deux variantes principales de ce procédé. Selon la première variante, la synthèse peptidique précède la fonctionnalisation. Selon la deuxième variante, la synthèse peptidique suit la fonctionnalisation. La deuxième variante permet d'obtenir un rendement plus important et est plus simple à mettre en oeuvre à l'échelle industrielle.

Selon la première variante, la première étape (étape de synthèse peptidique) permet d'obtenir le polypeptide de formule :

(IX) X₁-OH

Cette étape de synthèse peptidique peut être effectuée selon toute méthode connue de l'homme du métier. Elle peut en particulier être effectuée en phase liquide ou, de préférence, en phase solide.

De manière schématique, la synthèse peptidique comprend une succession de couplages d'acides aminés à partir d'une amorce (acide aminé initial ou fragment peptidique résultant des additions d'acides aminés précédentes) et de déprotections. Plus précisément, la synthèse peptidique peut successivement comprendre :
(a) la fourniture d'un fragment peptidique présentant une extrémité N-terminale non protégée, et d'un acide aminé protégé à son extrémité N-terminale ;
(b) l'établissement d'une liaison peptidique entre l'acide aminé et le fragment peptidique, à l'extrémité N-terminale dudit fragment peptidique ;
(c) la déprotection de l'extrémité N-terminale de l'acide aminé lié, pour fournir le fragment peptidique de l'étape (a) suivante.

Dans le cas de la synthèse peptidique en phase solide, le fragment peptidique (amorce) est lié à un support solide à son extrémité C-terminale. Le support solide est de préférence un polymère sous forme de particules (billes) insolubles ou solubles. On peut par exemple utiliser des résines à base de polystyrène, polyacrylamide, polyéthylèneglycol, cellulose, polyéthylène, polyester, latex, polyamide, polydiméthylacrylamide et des résines dérivées de celles-ci. Il est également possible d'utiliser un gel de silice ou des billes de verre à titre de support solide.

La liaison entre le fragment peptidique et le support solide est effectuée par l'intermédiaire d'un groupement fonctionnel approprié, dit lieur (ou « linker »). Ainsi, dans un premier temps l'acide aminé correspondant à l'extrémité C-terminale du polypeptide à synthétiser est fixé sur les groupements fonctionnels lieurs du support solide (en protégeant la fonction amine de l'acide aminé lors du couplage puis en la déprotégeant pour la rendre disponible pour la réaction suivante), ce qui constitue la première amorce, puis les acides aminés suivants sont ajoutés selon la succession de réactions rappelée ci-dessus.

Au cours des différentes réactions de couplage, il est avantageux d'utiliser un composé activateur, notamment un carbodiimide (par exemple dicyclohexylcarbodümide ou d iisopropylcarbod iim ide) en présence d'un triazole (par exemple 1-hydroxy-benzotriazole ou 1-hydroxy-7-azabenzotriazole), ou un sel phosphonium ou uronium d'un anion non-nucléophile (par exemple HBTU, HATU, TBTU ou PyBOP) ; ou encore d'utiliser des acides aminés activés sous forme d'halogénures d'acide, par exemple de fluorures d'acide.

Dans le cas d'une synthèse en phase solide, et une fois la dernière réaction de couplage d'acide aminé effectuée, on prévoit une réaction de séparation (ou clivage) du polypeptide de son support solide.

Au cours des différentes réactions de couplage, les extrémités N-terminales des acides aminés sont avantageusement protégées par un groupement protecteur, de préférence un groupement Fmoc (9H-fluorèn-9-ylméthoxycarbonyle) ou encore un groupement t-Boc (tert-butoxycarbonyle) ou NSC (2-(4-nitrophénylsulfonyl)éthoxycarbonyle).

De même, les chaînes latérales des résidus d'acides aminés sont de préférence protégées au cours des différentes réactions de couplage par un ou plusieurs groupements protecteurs appropriés, par exemple un groupement tert-butyle pour les chaînes portant une fonction carboxylique.

Dans ce cas, une fois la dernière réaction de couplage d'acide aminé effectuée, on peut prévoir une réaction de déprotection des chaînes latérales. Il faut toutefois noter qu'il peut être préférable de conserver l'ensemble des protections (à l'exception d'une éventuelle protection de la fonction COOH à l'extrémité C) en vue de l'étape de fonctionnalisation.

A l'issue de l'étape de synthèse peptidique, le polypeptide de formule (IX) est fonctionnalisé.

L'étape de fonctionnalisation comprend successivement :
- Eventuellement la protection de l'extrémité N-terminale du polypeptide de formule (IX) avec un groupement protecteur, de préférence choisi parmi la famille des carbamates, des amides ou des groupements alkyles, et notamment un groupement tert-butoxycarbonyle (t-Boc), Fmoc (9H-fluorèn-9-ylméthoxycarbonyle), trifluoroacétyle ou triphénylméthyle.
- Eventuellement également, la protection des fonctions des chaînes latérales des résidus d'acides aminés du polypeptide de formule (IX), et tout particulièrement des fonctions amines (de préférence au moyen des groupements protecteurs ci-dessus) et des fonctions carboxyliques (au moyen par exemple de groupements tert-butyles).
- Alternativement, et selon un mode de réalisation plus simple, on peut fournir le polypeptide de formule (IX) directement sous une forme dans laquelle l'ensemble des fonctions amines et carboxyliques sont protégées, en prévoyant une déprotection sélective de la fonction COOH de l'extrémité C-terminale.
- Le couplage du polypeptide de formule (IX) avec un composé amine de formule :

   (VIII) NH(CH₂-CH₂-S-G₁)₂

   G₁ étant un groupement protecteur.
- Eventuellement la déprotection du polypeptide.

En ce qui concerne le composé amine de formule (VIII), G₁ est choisi de préférence parmi les groupements fournissant une fonction thioéther, thioester (par exemple acétyle) ou disulfure (par exemple tert-butylsulfényle). De manière plus particulièrement préférée, G₁ est le groupement triphénylméthyle. Dans ce cas, le composé amine est la bis({2-[triphénylméthyl)sulfanyl]éthyl})amine. En ce qui concerne une méthode de préparation du composé amine de formule (VIII), on pourra se référer à l'exemple 1 ci-dessous.

Un activateur est avantageusement présent lors de la réaction de couplage, par exemple l'hexafluorophosphate de benzotriazol-1-yl-oxytripyrrolidinophosphonium (PyBOP) ou l'hexafluorophosphate de bromo-trispyrrolidinophosphonium (PyBROP) ou l'acide aminé C-terminal lui-même activé sous la forme d'un dérivé halogéné (en particulier sous la forme d'un fluorure d'acide aminé ou chlorure d'acide aminé), celui-ci pouvant être préformé ou formé in situ à l'aide de réactifs appropriés connus de l'homme de l'art. Parmi les halogénures d'acides aminés, les fluorures d'acides aminé sont préférés, préformés par réaction avec la 1,3,5-trifluorotriazine ou formés in situ à l'aide de TFFH (tétraméthylfluoroformamidinium hexafluorophosphate).

De manière générale, on peut également envisager tout réactif permettant l'activation de la fonction acide carboxylique de l'acide aminé connu de l'homme de l'art comme l'HBTU, le TBTU, l'HATU, le BOP... (on pourra se rapporter par exemple à Chemical approaches to the synthesis of peptides and proteins par Lloyd-Williams, P., Albericio, F., Giralt, E., 1997, CRC Press). Le PyBOP, le PyBROP, le BOP ou de façon plus générale les phosphoniums sont préférés.

A l'issue de l'étape de fonctionnalisation, le polypeptide de formule (I) est obtenu. Il est avantageux de prévoir à ce stade une étape de purification du composé, par exemple par chromatographie en phase liquide.

Selon la deuxième variante du procédé de production d'un polypeptide de formule (I), l'étape de fonctionnalisation précède l'étape de synthèse peptidique. Cette deuxième variante est mise en oeuvre en phase solide. Ainsi, l'étape de fonctionnalisation consiste dans ce mode de réalisation à créer un support solide amorce à partir d'un support solide préalablement fonctionnalisé.

A titre de support solide, on utilise un polymère soluble ou insoluble, le polymère insoluble étant de préférence sous forme de particules (billes). On peut par exemple utiliser une résine à base de polystyrène (de préférence) ou encore à base de polyacrylamide, polyéthylèneglycol, cellulose, polyéthylène, polyester, latex, polyamide, polydiméthylacrylamide, copolymère polyéthylèneglycol-polystyrène, copolymère polyéthylèneglycol-polyacrylamide ou encore une résine dérivée de celles-ci.

En outre, le support solide présente des groupements lieurs qui sont de préférence des groupements chloro-triphénylméthyle (ou chlorotrityle), où le triphénylméthyle est éventuellement substitué par un ou plusieurs substituants, notamment choisis parmi les substituants chlore, méthoxy, méthyle, fluor et cyano.

A titre d'exemples de support solide, on peut citer les résines polystyrènes présentant des groupements lieurs chlorure de trityle, chlorure de 2-chlorotrityle, chlorure de 4-méthyltrityle ou chlorure de 4-méthoxytrityle. De tels supports solides sont disponibles commercialement, par exemple auprès de Glycopep.

Selon un autre mode de réalisation, le support solide présente des groupements lieurs qui sont des groupements de type trityl-alcool, c'est à dire des groupements OH-Trt-CO-NH-, où le triphénylméthyle (Trt) est éventuellement substitué par un ou plusieurs substituants, notamment choisis parmi les substituants chlore, méthoxy, méthyle, fluor et cyano. Des supports solides présentant de tels groupements lieurs ont été décrits par exemple dans Quibell, JACS 1995, 117, 11656-11668, et sont disponibles commercialement, par exemple dans la gamme ChemMatrix® de supports solides polyéthylèneglycol.

L'utilisation de supports solides de ce type nécessite une étape préliminaire d'activation du support solide :
- soit avec un agent bromé (notamment bromure d'acétyle) pour modifier les groupements lieurs sous la forme Br-Trt-CO-NH- ;
- soit avec un agent chloré (notamment chlorure d'oxalyle) pour modifier les groupements lieurs sous la forme Cl-Trt-CO-NH-.

Une étape d'activation de ce type est décrite par exemple dans Harre et al., Reactive & functional polymers 1999, 41, 111-114.

Alternativement, on peut faire réagir directement la résine avec l'amine NH(CH₂-CH₂-SH)₂ en présence de BF₃.Et₂O, selon Singh, S. et al., J. Org. Chem. 2004, 69, 4551-4554.

L'utilisation de supports solides de ce type peut permettre d'utiliser des squelettes de résine de type polyéthylèneglycol ou polyéthylèneglycol-polystyrène, plus adaptés à la préparation de longs polypeptides que les résines de type polystyrène.

De préférence, les particules présentent un Dv50 compris entre 1 et 1000 µm. Le Dv50 est défini comme étant le 50^{ème} centile de la distribution de taille des particules, c'est-à-dire que 50 % des particules ont une taille inférieure au Dv50 et 50 % ont une taille supérieure au Dv50. De manière générale, le Dv50 est caractéristique du profil granulométrique (distribution volumétrique) des particules, et il peut être déterminé par granulométrie laser (pour une taille inférieure à 200 µm), ou par tamisage (pour une taille supérieure à 200 µm).

La préparation du support solide fonctionnalisé s'effectue en couplant le composé amine de formule :

(VIII') NH(CH₂-CH₂-SH)₂

au support solide ci-dessus. Le composé amine (VIII') peut lui-même être obtenu par déprotection du composé amine de formule (VIII) ci-dessus, dans lequel G₁ est un groupement protecteur.

Le couplage s'effectue en milieu acide afin de limiter les risques de démasquage de l'amine secondaire, ce qui induirait des réactions secondaires.

Les groupements chlorure de trityle en excès sont de préférence neutralisés, par exemple avec du méthanol. Il est alors important d'ajouter une base pour neutraliser le HCl formé.

On peut prévoir que la préparation du support solide fonctionnalisé fasse partie intégrante de la deuxième variante du procédé de fabrication des polypeptides de formule (I). Alternativement, le support solide fonctionnalisé peut être préparé à l'avance et de manière distincte, afin d'être prêt à l'emploi dans le cadre de la deuxième variante du procédé de fabrication des polypeptides de formule (I).

Le support solide fonctionnalisé est un support de résine polymère qui comprend un squelette principal (de type polystyrène, polyéthylèneglycol-polystyrène, polyacrylamide, polyéthylèneglycol, cellulose, polyéthylène, polyester, latex, polyamide, polydiméthylacrylamide, copolymère polyéthylèneglycol-polystyrène, copolymère polyéthylèneglycolpolyacrylamide ou dérivé de ceux-ci, selon le cas) et des groupements NH-(CH₂CH₂-S-Trt-)₂ liés au squelette principal par les deux groupements Trt, ou bien des groupements NH-(CH₂CH₂-S-Trt-CO-NH-)₂, liés au squelette principal par les deux fonctions NH, dans le cas où le support solide de départ est de type trityl-alcool.

Dans ce qui précède, Trt représente un groupement triphénylméthyle éventuellement substitué par un ou plusieurs substituants notamment choisis parmi les substituants chlore, méthoxy, méthyle, fluor et cyano .

Ainsi, ce support de résine polymère est essentiellement dépourvu de fonctions thiols libres, et présente des fonctions amines secondaires (fonctions disulfanylethylamines).

Par la suite, un acide aminé est couplé au support solide fonctionnalisé.

De préférence l'acide aminé est protégé à son extrémité N-terminale par un groupement protecteur labile en présence de base, de préférence choisi parmi le groupement 2-(4-nitrophénylsulfonyl)éthoxycarbonyl (NSC) ou Fmoc.

De préférence, l'acide aminé comporte des groupements protecteurs sur la totalité ou une partie (de préférence la totalité) des fonctions présentes sur sa chaîne latérale, et notamment les fonctions carboxylique, amine, alcool, phénol, guanidine (pour l'arginine) et imidazole (pour l'histidine). De tels groupements protecteurs sont connus de l'homme du métier. On pourra se reporter par exemple à l'ouvrage de référence Protective groups in organic synthesis, 2ème édition, T. Greene et P. Wuts, John Wiley & Sons, Inc.

De préférence, l'acide aminé est activé en présence de PyBOP ou de BOP ou de manière plus particulièrement préférée en présence de PyBROP, ou encore sous forme d'un halogénure, notamment fluorure (c'est-à-dire qu'un atome de fluor est lié au groupement acyle du résidu d'acide aminé).

L'acide aminé réagit avec la fonction amine secondaire présente sur le support solide fonctionnalisé pour former une liaison amide. Après couplage de l'acide aminé, on peut éventuellement procéder à la déprotection de celui-ci.

Par conséquent, à l'issue de l'étape de fonctionnalisation, on obtient un support de résine polymère, dit support amorce, comprenant des groupements G₂-NH-CHR-CO-N(CH₂CH₂-S-Trt-)₂ (R représentant une chaîne latérale d'acide aminé), que l'on peut également noter G₂-AA-N(CH₂CH₂-S-Trt-)₂ (AA représentant un résidu d'acide aminé), ces groupements étant liés au squelette principal par les deux groupements Trt ; ou bien des groupements G₂-AA-N-(CH₂CH₂-S-Trt-CO-NH-)₂, liés au squelette principal par les deux fonctions NH.

On rappelle que Trt désigne un groupement triphénylméthyle éventuellement substitué par un ou plusieurs substituants notamment choisis parmi les substituants chlore, méthoxy, méthyle, fluor et cyano. Par ailleurs, G₂ représente un atome d'hydrogène ou un groupement protecteur de fonction amine, selon que le groupement amine alpha de l'acide aminé a été déprotégé ou non. Enfin, les fonctions de la chaîne latérale R du résidu d'acide aminé AA peuvent être avantageusement protégées, comme mentionné ci-dessus.

Le support amorce ainsi obtenu (avec ou sans les groupements protecteurs) est également un objet de l'invention en tant que tel.

Puis l'étape de synthèse peptidique est effectuée, de manière analogue à ce qui a été décrit ci-dessus en relation avec le premier mode de réalisation, l'amorce initiale étant fournie par l'acide aminé couplé au support activé.

Une fois la dernière réaction de couplage d'acide aminé effectuée, on prévoit une réaction de séparation (ou clivage) du polypeptide de son support solide et si nécessaire les déprotections adéquates, après quoi on obtient le polypeptide de formule (I). Tout comme pour la première variante, il est avantageux de prévoir à ce stade une étape de purification du composé, par exemple par chromatographie en phase liquide.

### Procédé de fabrication des polypeptides de formule (I'), (V') et (XI')

Les polypeptides de formule (I'), (V') et (XI') mentionnés ci-dessus peuvent être obtenus très facilement à partir respectivement des polypeptides de formule (I), (V) et (XI) par oxydation à l'air, par exemple dans un tampon bicarbonate d'ammonium à environ pH 8. Une autre possibilité avantageuse consiste à utiliser de l'iode I₂ ou le diamide H₂NCO-N=N-CONH₂.

### Applications

Les polypeptides de formule (I) obtenus selon l'invention peuvent être utilisés pour produire une banque de polypeptides, par exemple à des fins de criblage.

Ils peuvent également être utilisés pour la fabrication de compositions pharmaceutiques, en combinaison avec un ou plusieurs adjuvants pharmaceutiquement acceptables (y compris un ou plusieurs véhicules pharmaceutiquement acceptables). A titre d'exemple de compositions pharmaceutiques pouvant être obtenues selon l'invention, on peut citer les médicaments et les préparations vaccinales.

Ils peuvent également être utilisés pour la réalisation de kits de diagnostic.

### EXEMPLES

Les exemples suivants illustrent l'invention sans la limiter.

L'exemple 1 concerne la préparation du composé bis({2-[triphénylméthyl)sulfanyl]éthyl})amine (composé de formule (VIII)).

Les exemples 2 et 3 conduisent à la fabrication d'un polypeptide 1 a de formule H-GFGQGFGG-N(CH₂CH₂SH)₂ (SEQ ID NO : 1, répondant à la formule générale (I) ci-dessus) selon la première variante du procédé de fabrication d'un polypeptide de formule (I), décrite ci-dessus (synthèse en phase liquide).

Les exemples 4 et 5 conduisent à la fabrication d'une résine fonctionnalisée, présentant des fonctions disulfanyléthylamine.

L'exemple 6 concerne la préparation d'un support dit « amorce » à partir de la résine fonctionnalisée précédente, sur laquelle est fixé un premier acide aminé.

L'exemple 7 concerne la préparation des polypeptides 1c (H-ILKEPVHGG-N(CH₂CH₂SH)₂) (SEQ ID No : 2), 1d (H-ILKEPVHGA-N(CH₂CH₂SH)₂) (SEQ ID NO : 3), 1e (H-ILKEPVHGV-N(CH₂CH₂SH)₂) (SEQ ID NO : 4) et 1f (H-ILKEPVHGY-N(CH₂CH₂SH)₂) (SEQ ID NO : 5), composés répondant à la formule générale (I) ci-dessus, selon la deuxième variante du procédé de fabrication d'un polypeptide de formule (I), décrite ci-dessus (synthèse en phase solide).

L'exemple 8 concerne la ligation des polypeptides respectifs 1 c, 1d et 1f avec un polypeptide 2 de formule H-CILKEPVHGV-NH₂ (SEQ ID NO : 6) répondant à la formule générale (II), pour fournir des polypeptides respectifs 3c (SEQ ID NO : 9), 3d (SEQ ID NO : 10) et 3f (SEQ ID NO : 11).

L'exemple 9 concerne la synthèse du polypeptide 1g (H-CHHLEPGG-N(CH₂CH₂SH)₂) (SEQ ID NO : 7), composé répondant à la formule générale (XI) ci-dessus, selon la deuxième variante du procédé de fabrication d'un polypeptide de formule générale (XI), décrite ci-dessus (synthèse en phase solide) ; ainsi que la cyclisation de ce polypeptide pour fournir un polypeptide cyclique répondant à la formule générale (X) ci-dessus.

L'exemple 10 concerne l'oxydation des polypeptides 1 c, 1 d, 1 e et 1f en dithiazépanes (composés répondant à la formule générale (I') ci-dessus).

L'exemple 11 concerne la ligation du polypeptide 1c avec un polypeptide 4 (SEQ ID NO : 17) comportant une homocystéine N-terminale, pour fournir un polypeptide 5 (SEQ ID NO : 18).

Les exemples 12, 13 et 14 concernent respectivement la synthèse d'un polypeptide 6 (SEQ ID NO : 19), d'un polypeptide 7 (SEQ ID NO : 20) et d'un polypeptide 8 (SEQ ID NO : 21).

L'exemple 15 concerne la ligation du polypeptide 7 et du polypeptide 8 pour former un polypeptide 7-8 (SEQ ID NO : 22), et l'exemple 16 concerne la ligation du polypeptide 6 avec le polypeptide 7-8 pour former un polypeptide 6-7-8 (SEQ ID NO : 23) après déprotection de la thiazolidine présente sur la cystéine N-terminale du fragment 7-8. Il s'agit donc d'une construction avec deux ligations successives. Le polypeptide 6-7-8 final est un polypeptide linéaire de séquence : H-IRNCIIGKGRSYKGTVSITKSGI KCQPWSSMIPHEHSFLPSSYRGKDLQENYCRNPRGEEGGPWCFTSNPEV

RYEVCDIPQCSEV-NH₂, les cystéines indiquées en gras étant celles impliquées dans les ligations.

### Exemple 1 : préparation de la bis({2-[triphénylméthyl)sulfanyl]éthyl})amine

1,50 g de bis(2-chloroéthyl)amine (8,4 mmol) et 4,65 g de triphénylméthanethiol (2 équivalents, 16,80 mmoles) sont introduits dans un ballon et placés sous atmosphère inerte. Sous agitation magnétique, 25 mL de diméthylformamide (DMF) anhydre sont ajoutés et le mélange réactionnel est refroidi dans un bain de glace. 4 équivalents de 1,8-diazabicyclo[5,4,0]undec-7-ène (DBU) sont additionnés goutte à goutte au mélange. Le mélange est laissé sous agitation à température ambiante durant 3 heures et la réaction est suivie par chromatographie sur couche mince (CCM) (éluant: cyclohexane / acétate d'éthyle / triéthylamine: 8 / 2 / 0,1). Passé ce délai, le solvant est évaporé à l'évaporateur rotatif. Le solide blanc obtenu est alors dissous dans 50 mL de dichlorométhane (DCM) et le produit est extrait trois fois avec une solution aqueuse de KH₂PO₄ à 5 %. Le produit est alors purifié par chromatographie sur colonne de gel de silice (éluant : cyclohexane / AcOEt / triéthylamine (TEA) : 8 / 2 / 0,1) pour obtenir 1,46 g de solide amorphe blanc (rendement de 28 %)

L'analyse du produit est la suivante.

Rf = 0,37 (silica gel, cyclohexane / AcOEt / TEA : 8 / 2 / 0,1); ¹H RMN (300 MHz, CDCl₃) δ 7,41 -7,37 (m, 12H, Trt), 7,15 - 7,29 (m, 18H, Trt), 2,23 - 2,36 (m, 8H, CH₂), 1,26 (s, 1 H, NH) ; ¹³C (75 MHz, CDCl₃) 154,1 ; 129,8 ; 128,1 ; 126,9 ; 47,9 ; 32,6 ; MALDI-TOF: 243,1 [Trt⁺], 622,3 [M+H⁺]⁺, 644,3 [M⁺+Na⁺].

### Exemple 2 : synthèse du polypeptide H-GFGQGFGG-OH (SEQ ID NO : 8)

1 g de résine Wang (charge : 1,1 mmoles/g) est placé dans un réacteur et solvaté 30 min dans le DMF. Parallèlement, dans un ballon placé sous atmosphère inerte, 3,27 g de Fmoc-Gly-OH (10 équivalents, 11 mmoles) sont dissous dans 100 mL d'un mélange dichlorométhane anhydre / DMF (99/1, v/v). Une solution de 857 µL de diisopropylcarbodiimide (5 équivalents, 5,50 moles) dans 5 mL de dichlorométhane anhydre est ajoutée, sous atmosphère inerte, sur la solution d'acide aminé à 0°C. Le mélange réactionnel est laissé sous agitation à cette température pendant 30 min. Passé ce délai, le solvant est évaporé et le solide blanc obtenu est dissous dans 5 mL de DMF. La solution est ajoutée à la résine avec 0,1 équivalent de DMAP (12,2 mg, 0,1 mmol) dans 1 mL de DMF, puis le mélange réactionnel est mis sous agitation 1 heure. La résine est alors filtrée, lavée avec 5 mL de DMF, 5 mL de dichlorométhane puis séchée sous vide. La charge finale de la résine (0,95 mmole/g ; 86%) est déterminée par la quantification UV de l'adduit dibenzofulvène-pipéridine libéré lors de la déprotection d'aliquots avec une solution à 20% de pipéridine dans le DMF.

La synthèse de polypeptides est effectuée en phase solide en utilisant la stratégie Fmoc/tert-butyl sur la résine Fmoc-Gly-Wang (échelle de 0,5 mmoles) préparée comme précédemment sur un synthétiseur de peptide à micro-ondes (CEM µ WAVES, Saclay, France). Le couplage est effectué en utilisant un excès 5 fois molaire de chaque acide aminé, l'activateur HBTU (O-benzotriazole-N,N,N',N'-tétraméthyluronium hexafluorophosphate) est utilisé avec un excès de 4,5 fois molaire et la base DiEA (diisopropyléthylamine) est utilisée avec un excès 10 fois molaire. La déprotection finale et le clivage du polypeptide de la résine sont réalisés avec 30 mL d'un mélange TFA (acide trifluoroacétique) / TIS (triisopropylsilane) / H₂O (95 / 2,5 / 2,5 en volume) pendant 1 heure. Le polypeptide est ensuite obtenu par précipitation dans un mélange éther diéthylique / heptane (1 / 1 en volume), dissout dans H₂O puis lyophilisé. La pureté du polypeptide (79%) est déterminée par HPLC (chromatographie en phase liquide) et électrophorèse capillaire. L'analyse LC-MS du polypeptide majoritaire concorde avec la structure du polypeptide attendu (C₃₃H₄₃N₉O₁₀ calculé 726,32 Da [M+H]⁺, observé 726,50 Da).

### Exemple 3: synthèse du polypeptide 1a (H-GFGQGFGG-N(CH₂CH₂SH)₂) (SEQ ID NO : 1) en phase liquide

102 mg de polypeptide H-GFGQGFGG-OH (SEQ ID NO : 8) (0,14 mmoles) sont dissous dans un minimum de diméthylformamide (DMF) anhydre et 39 µL de triéthylamine (TEA) (2 équivalents, 0,28 mmoles) sont additionnés à la solution. Sous agitation, 42 µL de di-tert-butyldicarbonate (Boc₂O) (1,3 équivalents, 0,18 mmoles) sont ajoutés au milieu réactionnel et la solution est homogénéisée par l'ajout de DMF anhydre. Le suivi de la réaction est effectué par HPLC.

174,2 mg de bis({2-[triphénylméthyl)sulfanyl]éthyl})amine (2 équivalents, 0,28 mmoles) sont ajoutés au milieu réactionnel précédent. 32 µL de DiEA (1,3 équivalents, 0,18 mmoles) ainsi que 94,7 mg d'hexafluorophosphate de benzotriazol-1-yl-oxytripyrrolidinophosphonium (PyBOP) (1,3 équivalents, 0,18 mmoles) sont ajoutés au mélange réactionnel. Le suivi du couplage est effectué par HPLC. Le polypeptide issu de cette étape de couplage est alors précipité et lavé dans un large volume d'éther diéthylique froid. Le polypeptide est alors dissous dans un minimum de DMF puis précipité et lavé une deuxième fois dans l'éther diéthylique.

12,2 mL d'un mélange TFA / TIS / eau (95 / 2,5 / 2,5) sont ajoutés sur le polypeptide protégé. La solution prend immédiatement une couleur jaune et se décolore rapidement. Après 30 min, le polypeptide est précipité dans 300 mL d'un mélange éther diéthylique / heptane (1 / 1) froid. La solution est centrifugée puis le polypeptide est lavé deux fois avec le mélange ci-dessus. Le polypeptide est solubilisé dans l'eau, congelé puis lyophilisé. Le produit est alors purifié par HPLC préparative (gradient : 0 à 40% de tampon B en 40 min, débit : 6 mL/min). 60 mg de polypeptide purifié sont alors obtenus après lyophilisation (rendement total = 50%).

L'analyse du produit est la suivante.

RMN ¹H (500 MHz, DMF-d7).

Phe₂ (2,98, dd, IH, Hβ ; 3,23, dd, 1 H, Hβ ; 4,63, m, 1 H, Hα ; 7,19 - 7,33, m, 5H, H_{arom.} ;) ; Gln4 (1,98-2,15, m, 2H, Hβ ; 2,31, m, 2H, Hy; 4,37, dt, 1H, Hα ; 6,97, s, 1 H, NH₂ ; 7,57, s, 1 H, NH₂ ; 8,09, d, 1 H, NH) ; Phe₆ (2,98, dd, 1 H, Hβ ; 3,23, dd, 1 H, Hβ ; 4,63, m, 1 H, Hα); Gly_{1, 3, 5, 7, 8} (3,72 - 4,21, m, 10H, Hα ; 8,6, large s, 3H, NH₃⁺ term.).

Groupement bis(tritylmercaptoéthyl)amino : 2,22 (t, 1 H, SH) ; 2,42 (t, 1 H, SH) ; 2,68 (q, 2H, CH₂) ; 2,81 (q, 2H, CH₂) ; 3,5 (t, 2H, CH₂) ; 3,58 (t, 2H, CH₂).

RMN ¹³C (125 MHz, DMF-d7) : Phe₂ (39,7, Cβ ; 57,5, Cα ; C_{arom.} ; CO); Gln₄ (30,2, Cβ ; 34,1, Cy ; 55,9, Cα; CO) ; Phe₆ (39,7, Cβ ; 58,2, Cα ; C_{arom.} ; CO) ; Gly_{1, 3, 5, 7, 8} (Cα; CO).

Groupement bis(tritylmercaptoéthyl)amino : 24,0 ; 25,0 ; 52,0 ; 53,0.

Spectrométrie de masse: LC-MS C₃₇H₅₂N₁₀O₉S₂ [M+H]⁺ calculé 845,34 Da ; observé 845,42 Da.

### Exemple 4: déprotection de l'amine secondaire bis({2-[triphénylméthyl)sulfanyl]éthyl})amine

12,5 mL d'un mélange TFA / TIS (97,5 / 2,5) sont versés sur 77,75 mg de bis({2-[triphénylméthyl)sulfanyl]éthyl})amine (0,125 mmoles). Le mélange réactionnel est laissé sous agitation pendant 30 minutes. La solution est alors évaporée à sec à l'aide d'un évaporateur rotatif pour obtenir un solide blanc. Le solide obtenu (composé de formule (VIII')) est solubilisé dans le cyclohexane et évaporé à sec, l'opération est renouvelée deux fois.

### Exemple 5 : couplage de l'amine déprotégée sur la résine chlorotrityle

893 mg de résine (résine chlorure de trityle sur squelette copolymère styrène avec 1 % de divinylbenzène, 200-400 mesh, 1,4 mmol/g, commercialisée par Merck sous la référence 01-64-0074) sont introduits dans un réacteur (1,25 mmoles). Sous argon, l'amine de l'exemple 4 est solubilisée dans 5 mL de DMF anhydre et déposée sur la résine à l'aide d'une seringue étanche au gaz. Le réacteur est mis en agitation une nuit sous papier d'aluminium. 40,5 µL de méthanol (1 mmol) et 116,5 µL de lutidine (1 mmol) sont ajoutés sur la résine. Après 30 minutes de solvolyse, la résine est lavée avec 2x2 minutes de DMF, 2x2 minutes MeOH, 2x2 minutes DMF, 2x2minutes DIEA à 5 % dans le DMF et finalement 2x2 minutes DMF. Les tests colorimétriques chloranil et d'Ellman révèlent la présence d'une amine secondaire et l'absence de thiol libre sur la résine.

Le procédé d'obtention de la résine fonctionnalisée de l'exemple 5 correspond au schéma général suivant:

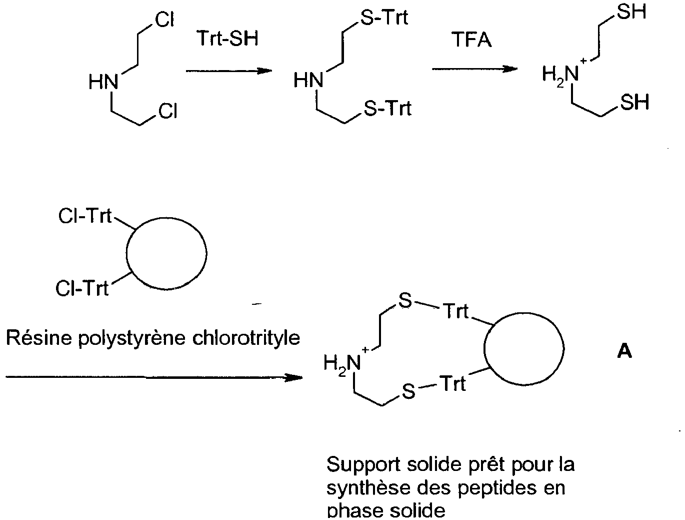

### Exemple 6: couplage d'acides aminés sur la résine fonctionnalisée de l'exemple 5 pour fournir des supports amorce (résine fonctionnalisée portant un premier acide aminé)

0,5 mmol de Fmoc-AA-F (acide aminé protégé par un groupe Fmoc et activé sous forme d'un fluorure d'acide) sont solubilisés dans 2 mL de DCM anhydre et ajoutés sur la résine de l'exemple 5 (0,125 mmol). On introduit ensuite 82,4 µL de N-méthylmorpholine (0,75 mmol). La réaction est agitée à température ambiante durant 2 heures. La résine est lavée ensuite avec 5x2 minutes de DCM anhydre et 3x2 minutes DMF. Les tests colorimétriques chloranil et d'Ellman montrent l'absence d'amine secondaire et de thiol libre sur la résine.

La charge finale de la résine est déterminée par un dosage UV-visible à 290 nm de l'adduit dibenzofulvène-pipéridine libéré lors de la déprotection avec une solution à 20% de pipéridine dans le DMF.

Cet exemple est mis en oeuvre avec 4 acides aminés différents : glycine, alanine, valine et tyrosine.

On obtient une charge de 0,15 mmol/g pour la glycine ; 0,124 mmol/g pour l'alanine ; 0,115 mmol/g pour la valine ; et 0,107 mmol/g pour la tyrosine.

Il faut noter que pour le couplage du premier acide aminé au support solide, d'autres agents de couplage peuvent être utilisés comme le PyBOP, le PyBrop, l'HBTU... Il a été constaté que le PyBrop donne les meilleurs résultats et permet l'utilisation directe des acides aminés sans nécessité de préactiver à l'aide d'un fluorure d'acide (ce qui est moins pratique expérimentalement).

### Exemple 7 : synthèse des polypeptides 1c (H-ILKEPVHGG-N(CH₂CH₂SH)₂), 1d (H-ILKEPVHGA-N(CH₂CH₂SH₂), 1e (H-ILKEPVHGV-N(CH₂CH₂SH)₂) et 1f (H-ILKEPVHGY-N(CH₂CH₂SH)₂) en phase solide

Le polypeptide 1c (SEQ ID NO : 2) est obtenu à partir du support amorce préparé dans l'exemple 6 avec la glycine.

Le polypeptide 1d (SEQ ID NO : 3) est obtenu à partir du support amorce préparé dans l'exemple 6 avec l'alanine.

Le polypeptide 1e (SEQ ID NO : 4) est obtenu à partir du support amorce préparé dans l'exemple 6 avec la valine.

Le polypeptide 1f (SEQ ID NO : 5) est obtenu à partir du support amorce préparé dans l'exemple 6 avec la tyrosine.

La synthèse des polypeptides peut être résumée comme suit:

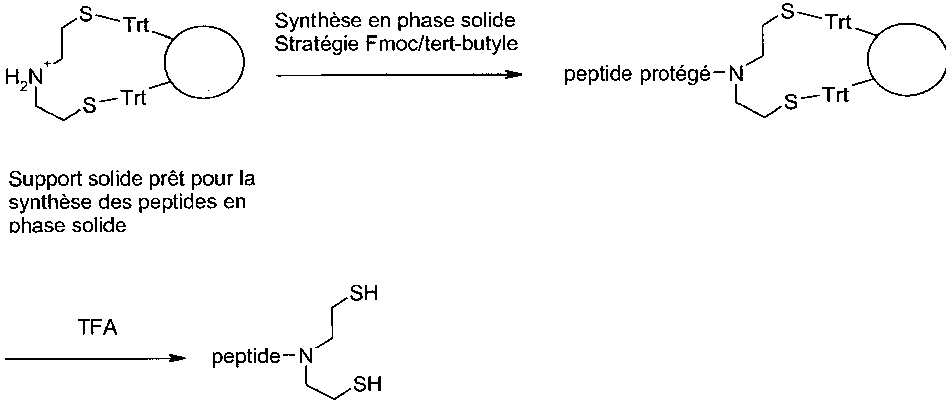

Les polypeptides obtenus dans l'exemple 7 ont la formule générique suivante : 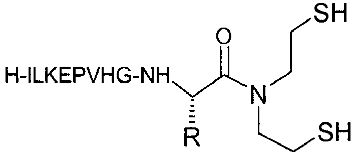
R=H1c
R=CH₃ 1d
R= CH(CH₃)₂ 1e
R= p-OHPhCH₂ 1f

La synthèse des différents polypeptides est effectuée en phase solide en utilisant la stratégie Fmoc/teri-butyle sur les résines respectives de l'exemple 6 (échelle de 0,1 mmol) sur un synthétiseur de peptide à micro-ondes (CEM µ WAVES, Saclay, France). Le couplage est effectué en utilisant un excès 5 fois molaire de chaque acide aminé, l'activateur HBTU est utilisé avec un excès de 4,5 fois molaire et la base DiEA est utilisée avec un excès 10 fois molaire.

La déprotection finale et le clivage du polypeptide de la résine sont réalisés avec 10 mL d'un mélange TFA/TIS/DMS/H₂O (92,5/2,5/2,5/2,5 en volume) pendant 1 heure. Le polypeptide est ensuite obtenu par précipitation dans 100 mL d'un mélange éther diéthylique/heptane (1/1 en volume), dissout dans H₂O puis lyophilisé.

La pureté de chaque polypeptide est déterminée par HPLC (91 % pour le polypeptide 1c avec une glycine, 83% pour le polypeptide 1 d, 80% pour 1e, et 88% pour 1f). L'analyse MALDI-Tof des polypeptides concorde avec la structure du polypeptide attendu (Peptide 1c C₄₇H₈₁N₁₃O₁₁S₂ [M+H]⁺ calculé 1068,6 Da, observé 1068,5. Polypeptide 1d C₄₈H₈₃N₁₃O₁₁S₂ [M+H]⁺ calculé 1082,6 Da, observé 1082,4. Polypeptide 1e C₅₀H₈₇N₁₃O₁₁S₂ [M+H]⁺ calculé 1110,6 Da, observé 1110,5). Polypeptide 1f C₅₄H₈₇N₁₃O₁₂S₂ [M+H]⁺ calculé 1174,6 Da, observé 1174,6).

La purification des polypeptides est réalisée sur colonne C18 nucléosil en acetonitrile-H₂O (80-20) en TFA, avec un gradient de 0 à 30 % en 30 mn pour le polypeptide 1c, et un gradient de 0 à 10 % en 5 mn puis 10 à 25% en 25 mn pour les polypeptides 1 d et 1 e.

La pureté déterminée par HPLC est de 96% pour le polypeptide 1c avec un rendement global de 35%, 97% pour le polypeptide 1d avec un rendement de 31%, et de 99% pour le polypeptide 1 e avec un rendement de 27%.

### Exemple 8 : ligation des polypeptides 1c, 1d et 1f avec le polypeptide 2 (H-CILKEPVHGV-NH₂)

Les ligations respectives des polypeptides 1 c, 1d et 1f obtenus à l'exemple 7 avec le polypeptide 2 (SEQ ID NO : 6) sont effectuées selon le schéma suivant :

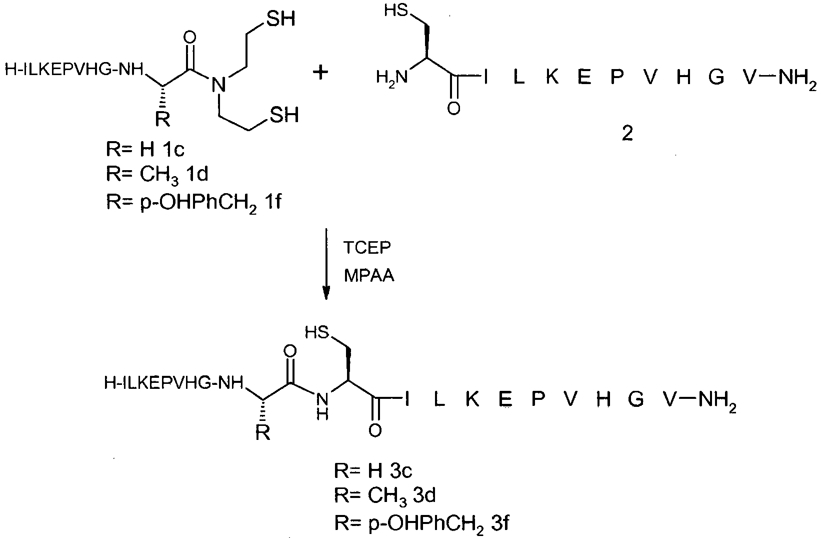

Ces ligations permettent d'obtenir les polypeptides respectifs 3c (de formule H-ILKEPVHGGCILKEPVHGV-NH₂, SEQ ID NO : 9), 3d (de formule H-ILKEPVHGACILKEPVHGV-NH₂, SEQ ID NO : 10) et 3f (de formule H-ILKEPVHGYCILKEPVHGV-NH₂, SEQ ID NO : 11).

Ligation du polypeptide 1c.

336 mg de MPAA (2 mmol, 200mM) et 234 mg de TCEP (800 µmol, 80 mM) sont dissous dans 10 mL de tampon phosphate 0,1 M (pH ajusté à 7,5). 10,2 mg de polypeptide 1c est dissous dans le mélange (7,2 µmol, 0,72 mM), cette solution est additionnée sur 15,3 mg de polypeptide 2 H-CILKEPVHGV-NH₂ (10,6 mmol, 1,06 mM). Le mélange est placé sous argon puis mis sous agitation à 37°C. Le produit est ensuite purifié par RP-HPLC pour donner 5,9 mg de polypeptide 3c (32%).

Ligation du polypeptide 1d.

Dans un premier temps, on prépare une solution MPAA/TCEP·HCl. 33,52 mg de MPAA et 57,54 mg de TCEP·HCl sont pesés dans un tube en polypropylène de 1,5 mL. 1 mL de tampon phosphate 0,1M à pH=7,3 puis 140 µL de NaOH à 6M sont ajoutés et entraînent la solubilisation complète des poudres. 20 µL NaOH à 6M supplémentaires sont ajoutés pour ajuster le pH de la solution à 7,05.

Pour la réaction de ligation proprement dite, 5,99 mg de polypeptide 1d et 9.05 mg polypeptide 2 sont pesés dans le même ballon de 5 mL en verre. 600 µL de la solution ci-dessus sont ajoutés. Le milieu réactionnel est mis sous argon par 3 cycles vide/argon, puis placé dans un bain d'huile thermostaté à 37°C et agité à l'aide d'un barreau magnétique.

Au bout de 26h30, le milieu réactionnel est transféré dans un tube en polypropylène de 15 mL. Le ballon réactionnel est rincé par 3,4 mL de tampon A (eau contenant 0.05% en volume de TFA) que l'on transfère dans le tube de 15 mL. 4 extractions (4 × 4 mL) sont réalisées avec de l'éther. On acidifie d'avantage la phase aqueuse par addition de 150 µL de TFA à 10% dans l'eau. 4 nouvelles extractions (4 × 4 mL) à l'éther sont renouvelées.

La phase aqueuse est alors injectée en HPLC préparative (température ambiante, 230 nm, colonne C18 nucleosil 120 A-5 µm, tampon A (eau contenant 0,05 % en volume de TFA), tampon B acétonitrile/eau 4/1 en volume contenant 0,05 % en volume de TFA, débit 3 mL/min, gradient 0 à 15% B en 15 min, puis 15 à 100 % B en 283 min, volume injecté 4 mL).

Après lyophilisation, on recueille 8,5 mg de produit de ligation (rendement=77%).

C₉₃H₁₅₆N₂₆O₂₃S [M+H]⁺ calculé 2038,16, trouvé 2038,07.

Détermination de la pureté énantiomérique pour l'alanine : 1,76% D-énantiomère.

Ligation du polypeptide 1 f.

Dans un premier temps, on prépare une solution MPAA/TCEP·HCl. 33,63 mg MPAA et 57,37 mg de TCEP·HCl sont pesés dans un tube en polypropylène de 1,5 mL. 1 mL de tampon phosphate 0,1 M pH=7,3 puis 140 µL de NaOH à 6M sont ajoutés et entraînent la solubilisation complète des poudres. Le pH de la solution est ajusté à 7,05 avec NaOH à 6M.

Pour la réaction de ligation proprement dite, 3,97 mg polypeptide 1 e et 5,75 mg de polypeptide 2 sont pesés dans le même ballon de 5 mL en verre. 374 µL de la solution ci-dessus sont ajoutés. Le milieu réactionnel est mis sous argon par 3 cycles vide/argon, puis placé dans un bain d'huile thermostaté à 37°C et agité à l'aide d'un barreau aimanté.

Au bout de 44h30, on rajoute 78,6 µL (30 eq) d'une solution de TCEP·HCl à 1 M dans le tampon phosphate réajusté à pH=7.0 à l'aide de soude 6N.

Au bout de 4 jours et demi, le milieu réactionnel est transféré dans un tube en polypropylène de 15 mL. Le ballon réactionnel est rincé par 3,5 mL de tampon A et acidifié avec 150 µL de TFA à 10% dans l'eau que l'on transfère également dans le tube. 3 extractions (3 × 5,5 mL) sont réalisées avec de l'éther.

La phase aqueuse est alors injectée en HPLC préparative (RT, 230 nm, colonne C18 nucleosil 120 A-5 µm, tampon A 100% eau contenant 0.05% TFA, tampon B acétonitrile/eau 4/1 contenant 0.05% TFA, débit 3 ml/min, gradient 0 à 15% B en 15 min, puis 15 à 100% B en 283 min, vol injecté 4 mL).

Après lyophilisation, on recueille 3,80 mg de produit de ligation (rendement=54%).

C₉₉H₁₆₀N₂₆O₂₄S [M+H]⁺ calculé 2130,18, trouvé 2130,2.

Détermination de la pureté énantiomérique de Tyr : 1,25% D-énantiomère.

Les **Fig. 1** à **3d** illustrent le suivi de la réaction de ligation pour la ligation des polypeptides 1 c, 1d et 1f.

### Exemple 9 : synthèse du polypeptide 1g (H-CHHLEPGG-N(CH₂CH₂SH)₂) en phase solide et cyclisation de ce polypeptide

Le polypeptide 1g (SEQ ID NO : 7) a été synthétisé comme le polypeptide 1 c.

La synthèse du polypeptide 1g est effectuée en phase solide en utilisant la stratégie Fmoc/tertButyle (échelle 50 µmol) sur un synthétiseur de peptides à micro-ondes. Le couplage est effectué en présence d'HTBU comme agent activant (4,5 éq) et de DIEA comme base (10 éq). A la fin de la synthèse la résine est lavée au dichlorométhane (2 x 5 mL), à l'éther éthylique (2 x 5 mL) puis séchée. La déprotection finale et le clivage du polypeptide sont réalisés avec 5 mL du mélange TFA/TIS/H2O/DMS, 9,25/0,25/0,25/0,25 en volume pendant une heure. Le polypeptide est précipité dans 50 mL d'un mélange éther/heptane (1/1), dissout dans l'eau puis lyophilisé.

Polypeptide 1g : C39H61N13O1053, analyse MALDI-TOF [M+H]+ calculée 968,19, observée 968,2.

Le polypeptide 1g est ensuite cyclisé pour fournir le polypeptide 3g, selon le schéma suivant :

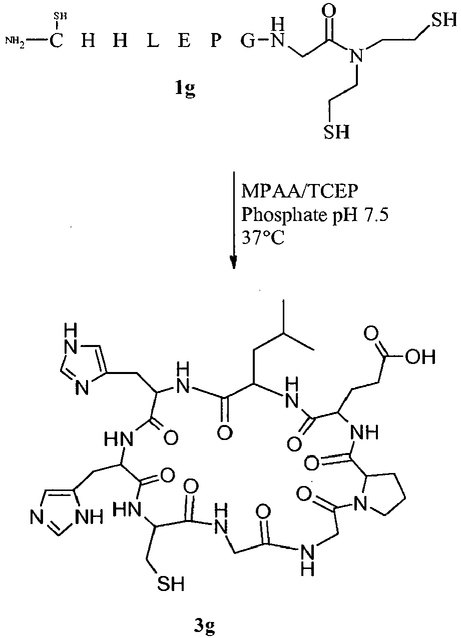

Les conditions utilisées pour la cyclisation sont identiques aux conditions utilisées pour liguer le polypeptide 1 c au polypeptide 2.

33,64 mg de MPAA (200 mM) et 22,9 mg de TCEP (80 mM) sont dissous dans 1 mL de tampon phosphate 0.1 M à pH 7.5 ajusté à l'aide d'une solution de NaOH 6N.

1,0 mg (0,76 µmol) de polypeptide 1g est dissous dans le mélange (764 µL, 1 mM), placé sous argon puis mis sous agitation à 37°C. La réaction conduit exclusivement à la formation du polypeptide cyclique 3g (SEQ ID NO : 12).

Polypeptide 3g : cyclo(CHHLEPGG) ; C₃₅H₄₉N₁₂O₁₀S analyse MALDI-TOF [M+H]+ calculé 831,1 observé 831,1.

### Exemple 10 : oxydation des polypeptides 1c, 1d, 1e et 1f en dithiazépanes (SEQ ID NO: 13, 14, 15 et 16)

Les polypeptides 1c, 1d, 1e et 1f sont tels qu'obtenus à l'exemple 7. Ces polypeptides sont oxydés selon le schéma général suivant :

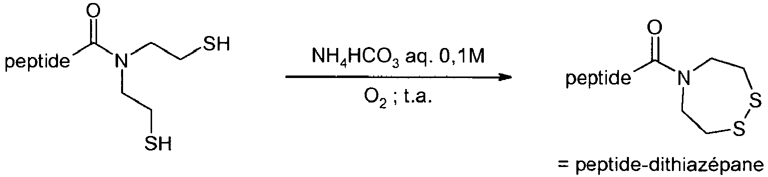

Chaque polypeptide est clivé du support solide par l'action d'une solution TFA/DMS/TiS/H₂O (92,5/2,5/2,5/2,5 ; v/v). Le polypeptide est alors précipité dans un large volume d'un mélange éther diéthylique/heptane (1/1 ; v/v) et lavé deux fois à l'aide de cette solution. Le polypeptide brut lyophilisé après l'étape de coupure est ensuite dissous dans une solution de bicarbonate d'ammonium à 0,1 M préalablement dégazée pendant 10 min par bullage d'azote (1 mg/mL).

Le mélange est alors laissé à température ambiante sous agitation forte. L'évolution de la réaction est suivie par spectrométrie de masse MALDI-TOF jusqu'à disparition totale de la forme réduite du polypeptide considéré. Le polypeptide est enfin purifié par RP-HPLC (gradient éluant A (H₂O/0,05% TFA)/éluant B (80% Acétonitrile/20% H₂O/0,05% TFA) : 0 à 10% en 10 min puis 10% à 25 % en 25 min) puis congelé et lyophilisé.

Le tableau ci-dessous résume les résultats obtenus (analyse MALDI-TOF).

| Polypeptide | m/z [M+H]⁺_{calc}. | M/z [M+H]⁺_{obs}. | Rendement final(%) |
|---|---|---|---|
| 1c | 1066,6 | 1066,6 | 17 |
| 1d | 1080,6 | 1080,6 | 13 |
| 1e | 1108,6 | 1108,6 | 23 |
| 1f | 1172,6 | 1172,6 | 20 |

### Exemple 11 : ligation entre le polypeptide 1c et le polypeptide 4

On effectue une ligation du polypeptide 1 c (SEQ, ID NO : 2) avec le polypeptide 4 (SEQ ID NO : 17) qui est identique au polypeptide 2 à ceci près que la cystéine N-terminale est remplacée par une homocystéine. Cette réaction permet d'obtenir le polypeptide 5 (SEQ ID NO : 18). Le schéma de la réaction est le suivant :

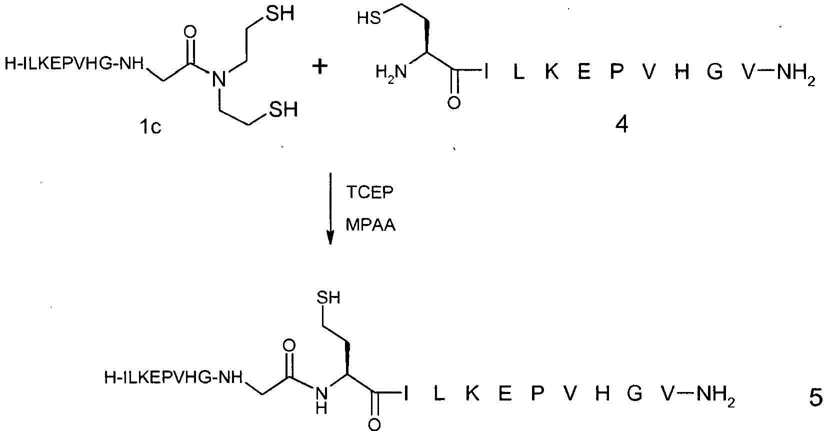

33,6 mg de MPAA (0,2 mmol, 200 mM) et 57,4 mg de TCEP (0,2 mmol, 200 mM) sont dissous dans 1 mL de tampon phosphate 0,1 M (pH ajusté à 7,35). 6,15 mg de polypeptide 1c (4,4 µmol, 7 mM) et 9,35 mg de polypeptide 4 (H-homoCyslLKEPVHGV-NH₂) (6.55 µmol, 10.5 mM) sont dissous avec le mélange (624 µL). Le mélange est placé sous argon puis mis sous agitation à 37°C. Le produit est ensuite purifié par RP-HPLC pour donner 3,3 mg de polypeptide 5 (29%). C₉₁ H₁₅₂ N_26 O₂₃ S. Analyse MS MALDI-TOF (monoisotopique) [M+H]⁺ 2010,12 calculé, 2009,5 trouvé.

### Exemple 12 : synthèse du polypeptide 6 (SEQ ID NO : 19)

Le polypeptide 6 présente la séquence suivante :

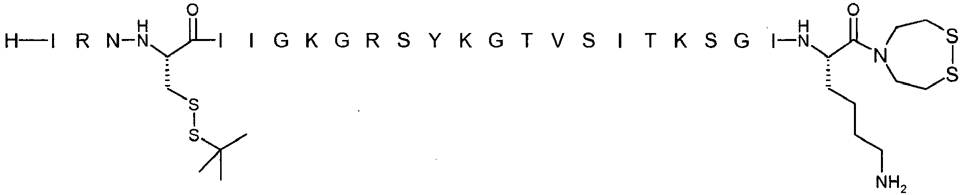

La résine décrite dans l'exemple 5 (0,5 mmol, 0,175 mmol/g) est conditionnée dans le dichlorométhane. Fmoc-Lys(Boc)-OH (2.342 g, 5 mmol) est dissous dans le dichlorométhane et quelques gouttes de DMF pour aider à la solubilisation puis ajouté sur la résine. PyBrop (2.331 g, 5 mmol) est dissous dans le minimum de dichlorométhane puis ajouté sur la résine. La DIEA (2.613 mL, 15 mmol) est ensuite additionnée sur la résine et le couplage dure 2h. La résine est ensuite lavée 3 x 2 minutes au dichlorométhane. La résine est ensuite traitée par 10% Ac₂O/5% DiEA/dichlorométhane (10 mL, 2 min) puis (10 mL, 20 min). La résine est ensuite lavée 5 x 2 minutes au dichlorométhane.

Le polypeptide 6 est assemblé sur une partie de la résine précédente (0,25 mmol, 0.175 mmol/g) avec un synthétiseur de peptides (CEM µWaves, Saclay, France), en utilisant la stratégie Fmoc/*tert*-butyle. Le couplage est effectué avec les acides aminés (0,2 M, 4 eq), l'activateur HBTU (0,5 M, 3,6 eq) et la base DIEA (2 M, 8 eq). La déprotection finale et le clivage du peptide de la résine sont réalisés avec TFA/TIS/DMS/thioanisole/H₂O (90/2,5/2,5/2,5/2,5 en volume, 25 mL) pendant 2h30. Le peptide est précipité dans un mélange froid éther diéthylique/heptane (1/1 en volume), dissout dans un minimum d'eau et lyophilisé. 288 mg de peptide brut sont obtenus (R=32%). C₁₂₀H₂₁₆N₃₆O₃₁S₄ LC-MS [M+H]⁺ calculé (masse moyenne) 2788.5; observé 2788.1.

Une partie du polypeptide 6 est ensuite oxydée avant purification. Pour cela, le polypeptide 6 (49,8 mg) est dissous dans AcOH/eau 4/1 (2 mL). Il est additionné goutte à goutte dans une solution d'iode dans AcOH/eau 4/1 (25 mL, « 10 eq »). Après 10 minutes d'agitation, le milieu réactionnel est transféré dans une ampoule à décanter contenant de l'eau (60 mL). 3 extractions à l'éther sont réalisées (3 x 60 mL). La phase aqueuse est congelée et lyophilisée pour donner 44.1 mg de peptide brut.

Après purification RP-HPLC (colonne Atlantis dC18 OBD 5 µm,19 x 100 mm, 210-300 nm, tampon A 100% eau contenant 0.05% TFA, tampon B CH₃CN/eau 4/1 contenant 0.05% TFA, gradient : 20 à 40% de tampon B en 40 min, débit 25 mL/min) on obtient 7.2 mg de peptide pur (R=14.5%) C₁₂₀H₂₁₄N₃₆O₃₁S₄ LC-MS [M+H]⁺ calculé (masse moyenne) 2786.52; observé 2786.33.

### Exemple 13 : synthèse du Polypeptide 7 (SEQ ID NO : 20)

Le polypeptide 7 présente la séquence suivante :

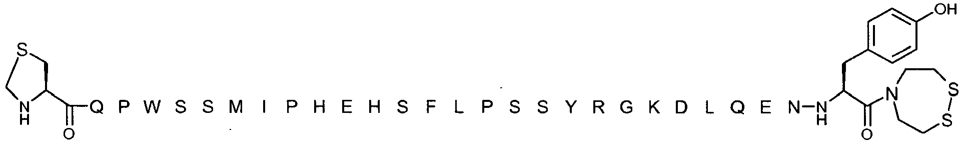

La résine décrite dans l'exemple 5 (0,5 mmol, 0,175 mmol/g) est conditionnée dans le dichlorométhane. Fmoc-Tyr-OH (2,298 g, 5 mmol) est dissous dans le dichlorométhane (<5 mL) et quelques gouttes de DMF pour aider à la solubilisation puis ajouté sur la résine. PyBrop (2,331 g, 5 mmol) est dissous dans le minimum de DCM puis ajouté sur la résine. DIEA (2,614 mL, 15 mmol) est ensuite additionné sur la résine et le couplage dure 2 h. La résine est ensuite lavée 4 x 2 minutes au dichlorométhane. La résine est ensuite traitée par 10% Ac₂O/5% DiEA/DCM (10 mL, 2 min) puis (10 mL, 20 min). La résine est ensuite lavée 5 x 2 minutes au dichlorométhane.

Le polypeptide 7 est assemblé sur la résine précédente (0,5 mmol, 0,175 mmol/g) avec un synthétiseur de peptides (CEM µWaves, Saclay, France), en utilisant la stratégie Fmoc/tert-butyle. Le couplage est effectué avec les acides aminés (0.2 M, 4 eq), l'activateur HBTU (0.5 M, 3.6 eq) et la base DIEA (2 M, 8 eq). Le solvant de lavage (DMF) ainsi que le solvant de Fmoc-Met-OH contiennent 1% de thioanisole afin d'éviter au maximum l'oxydation de la méthionine de la séquence.

La résine est séparée en 2 après la glutamine en position 2 (0,25 mmol) afin de coupler la Boc-L-Thz-OH manuellement. Pour ce faire, la résine est lavée 4 x 2 minutes au DMF, pesée en DMF et divisée en 2. HBTU (379,3 mg, 1 mmol) est dissous dans le DMF (1100 µL). HOBt (135 mg, 1 mmol) est dissous dans le DMF (500 µL) et additionné sur l'HBTU. Boc-L-Thz-OH (233,29 mg, 1 mmol) est dissous dans DMF (500 µL) et additionné au mélange HBTU/HOBt. DIEA (522,7 µL, 3 mmol) est ensuite ajouté sur le mélange. On agite pendant 1 minute puis le mélange est additionné sur la résine et le couplage dure 45 minutes. La résine est ensuite lavée 4 x 2 minutes au DMF, 4 x 2 minutes au dichlorométhane puis 3 x 2 minutes à Et₂O et séchée.

La déprotection finale et le clivage du peptide de la résine sont réalisés avec TFA/TIS/DMS/thioanisole/H₂O (90/2,5/2,5/2,5/2,5 en volume, 25 mL) pendant 2h30. Le peptide est précipité dans un mélange froid éther diéthylique/heptane (1/1 en volume), dissout dans un minimum d'eau et lyophilisé. 369 mg de peptide brut sont obtenus (R=37.6%). C₁₅₃H₂₂₃N₄₁O₄₄S₄ MALDI-TOF [M+H]⁺ calculé (résolution monoisotopique) 3467.54; observé 3466.0.

Une partie du polypeptide 7 est ensuite oxydée avant purification. Pour cela, le fragment 2 (51,8 mg) est dissous dans CH₃CN (2.38 mL) puis 0,2 M tampon phosphate pH=7.3 (9,.50 mL) sont ajoutés. Il est additionné goutte à goutte sur une solution de 10 mM diamide dans l'eau (1,32 mL, 1 eq). Après 15 minutes d'agitation, le milieu réactionnel est dilué avec du tampon A (1,8 mL) et injecté en RP-HPLC (colonne Atlantis dC18 OBD 5 µm,19 x 100 mm, 210-300 nm, tampon A 100% eau contenant 0.05% TFA, tampon B CH₃CN/eau 4/1 contenant 0,05% TFA, gradient : 25 à 45 % de tampon B en 40 min, débit 25 mL/min) on obtient 11,2 mg de peptide pur (R=21.6%) C₁₅₃H₂₂₁N₄₁O₄₄S₄ LC-MS [M+H]⁺ calculé (masse moyenne) 3467,97; 3467,38.

### Exemple 14 : synthèse du polypeptide 8 (SEQ ID NO : 21)

Le polypeptide 8 présente la séquence suivante :

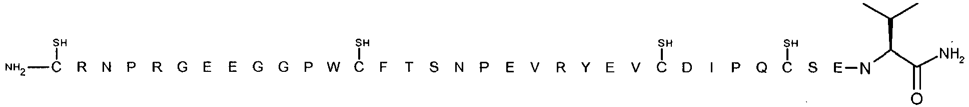

Le polypeptide 8 est assemblé sur la résine Novasyn TGR (0,5 mmol, 0,25 mmol/g) avec un synthétiseur de peptides (CEM µwaves, Saclay, France), en utilisant la stratégie Fmoc/*tert*-butyle. Le couplage est effectué avec les acides aminés (0,2 M, 4 eq), l'activateur HBTU (0,5 M, 3,6 eq) et la base DIEA (2 M, 8 eq). La déprotection finale et le clivage du peptide de la résine sont réalisés avec TFA/EDT/H₂O/TIS (94/2,5/2,5/1 en volume, 30 mL) pendant 2h30. Le peptide est précipité dans un mélange froid éther diéthylique/heptane (1/1 en volume), dissout dans un minimum d'eau et lyophilisé. Après purification RP-HPLC (colonne C18 vydac 50 cm x 2 cm, 280 nm, tampon A 100% eau contenant 0.05% TFA, tampon B CH₃CN/eau 4/1 contenant 0.05% TFA, gradient : 0 à 20 % de tampon B en 10 min puis 20 à 50 % de tampon B en 60 min, débit 30 mL/min) on obtient 272 mg de peptide pur (R=13%) C₁₅₈H₂₃₉N₄₇O₅₂S₄ MALDI-TOF [M+H]⁺ calculé (résolution monoisotopique) 3755,6 ; observé 3755,7.

### Exemple 15 : ligation du polypeptide 7 et du polypeptide 8 (en boîte à gants) pour obtenir le polypeptide 7-8 (SEQ ID NO : 22)

MPAA (33,70 mg) et TCEP.HCl (57,30 mg) sont dissous dans 0,1 M tampon phosphate pH=7,3 contenant 4 M guanidine HCl (1 mL). Le pH de la solution est réajusté à 7.2 avec de la soude 5N (200 µL). Les polypeptides 7 (8,5 mg) et 8 (18,25 mg, 2 eq) sont pesés dans le même tube et dissous avec la solution précédente (309 µL). Le milieu réactionnel est placé dans un bain à 37°C. Après 24 h, le milieu réactionnel est dilué par la même solution (300 µL).

25 h plus tard, 56 mM O-méthylhydroxylamine dans 0,1 M tampon acétate à pH=3,93 (1,52 mL) est ajouté. Le pH du milieu réactionnel est réajusté à pH=4,15 avec de l'acide acétique (35 µL). Après 20 h à 37°C, le milieu réactionnel est sorti de la boîte à gants. MPAA est extrait avec Et₂O (3 x 6 mL). Le milieu réactionnel est traité 20 minutes à TCEP.HCl (28 mg) avant purification par RP-HPLC (colonne uptisphère 5C4 27.5 cm x 1 cm, 215 nm, tampon A 100% eau contenant 0.05% TFA, tampon B CH₃CN/eau 4/1 contenant 0.05% TFA, gradient : 0 à 20 % de tampon B en 3 min puis 20 à 50 % de tampon B en 57 min, débit 6 mL/min). On obtient 4,4 mg de polypeptide 7-8 pur (R=25,4%) C₃₀₆H₄₅₁N₈₇O₉₆S₆ MALDI-TOF [M+H]⁺ calculé (masse moyenne) 7077,8; observé 7078,5.

### Exemple 16 : ligation du polypeptide 6 et du Polypeptide 7-8 (en boîte à gants) pour obtenir le Polypeptide 6-7-8 (SEQ ID NO : 23)

MPAA (33,74 mg) et TCEP.HCl (57,54 mg) sont dissous dans 0,1 M de tampon phosphate à pH=7,3 contenant 4 M de guanidine HCl (1 mL). Le pH de la solution est réajusté à 7,2 avec de la soude 5 N (220 µL). Les polypeptides 7-8 (3,85 mg) et 6 (2,89 mg, 1,7 eq) sont pesés dans le même tube et dissous avec la solution précédente (115 µL). Le milieu réactionnel est placé dans un bain à 37°C.

Après 24 h, on voit la formation du produit de ligation, à savoir le polypeptide 6-7-8 de séquence: H-IRNCIIGKGRSYKGTVSITKSG IKCQPWSSMIPHEHSFLPSSYRGKDLQENYCRNPRGEEGGPWCFTSNPEV RYEVCDIPQCSEV-NH₂

C₄₁₈H₆₄₈N₁₂₂O₁₂₇S₇ MALDI-TOF [M+H]⁺ calculé (masse moyenne) 9640,01; observé 9640,1.

## Revendications

1. Procédé de fabrication d'un polypeptide de formule :
(III) X₁-X"-X₂
X₁ et X₂ représentant chacun un fragment peptidique, et X" représentant un résidu d'acide aminé comportant une fonction thiol,
ledit procédé comprenant au moins une étape de réaction de ligation entre un polypeptide de formule :
(I) X₁-N(CH₂CH₂SH)₂
et un polypeptide de formule :
(II) H-X"-X₂.

2. Procédé selon la revendication 1, dans lequel la réaction de ligation est effectuée en mettant en contact un polypeptide de formule : 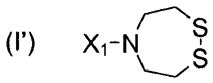
avec le polypeptide de formule (II), en présence d'au moins un composé réducteur des liaisons disulfure, le polypeptide de formule (I') étant réduit *in situ* en le polypeptide de formule (I) pour la réaction de ligation.

3. Procédé selon la revendication 1 ou 2, dans lequel X₂ représente un fragment peptidique de formule
(IV) X2'-(Xᵢ"-Xᵢ')_{i=3...n}
n étant un entier supérieur ou égal à 3, chaque Xᵢ", pour i entier compris entre 3 et n, représentant un résidu d'acide aminé comportant une fonction thiol, et chaque Xᵢ', pour i entier compris entre 2 et n, représentant un fragment peptidique ;
ledit procédé comprenant, avant l'étape de réaction de ligation entre le polypeptide de formule (I) et le polypeptide de formule (II), une succession de n-2 étapes de réaction de ligation, la j^{ème} étape de réaction de ligation, pour j entier compris entre 1 et n-2, étant une réaction de ligation entre un polypeptide de formule :
(V) H-X_{n-j"}-Xₙ₋ⱼ'- N(CH₂CH₂SH)₂
dans lequel la fonction amine et / ou la fonction thiol du résidu Xₙ₋ⱼ" est protégée
et un polypeptide de formule :
(VI) H-(Xᵢ"-Xᵢ')_{i=(n-j+1)...n}
pour former un polypeptide de formule :
(VII) H-(Xᵢ"-Xᵢ')_{i=(n-j)...n}
le polypeptide de formule (VII) subissant une déprotection de la fonction thiol du résidu Xₙ₋ⱼ" à l'issue de la réaction de ligation.

4. Procédé selon la revendication 3, dans lequel une ou plusieurs des n-2 étapes de réaction de ligation entre le polypeptide de formule (V) et le polypeptide de formule (VI) est effectuée en mettant en contact un polypeptide de formule : 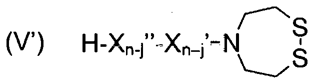 avec le polypeptide de formule :
(VI) H-(Xᵢ"-Xᵢ')_{i=(n-j+1)...n}
j étant un entier compris entre 1 et n-2, en présence d'au moins un composé réducteur des liaisons disulfure.

5. Procédé de fabrication d'un polypeptide cyclique de formule : 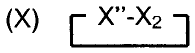
X₂ représentant un fragment peptidique, et X" représentant un résidu d'acide aminé comportant une fonction thiol,
ledit procédé comprenant au moins une étape de réaction de ligation d'un polypeptide de formule :
(XI) H-X"-X₂-N(CH₂CH₂SH)₂
avec lui-même.

6. Procédé selon la revendication 5, dans lequel la réaction de ligation est effectuée en mettant en contact un polypeptide de formule : 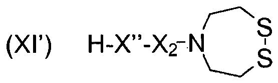
avec au moins un composé réducteur des liaisons disulfure, le polypeptide de formule (XI') étant réduit *in situ* en le polypeptide de formule (XI) pour la réaction de ligation.

7. Procédé selon l'une des revendications 1 à 6, dans lequel la ou les réactions de ligation sont effectuées en milieu aqueux, de préférence à un pH compris entre 6,5 et 8,5, de manière plus particulièrement préférée entre 7 et 8, et idéalement d'environ 7,5.

8. Procédé selon l'une des revendications 1 à 7, dans lequel la ou les réactions de ligation sont effectuées en présence d'au moins un composé réducteur des liaisons disulfure, de préférence choisi parmi la tris(2-carboxyéthyl)phosphine, l'acide 4-mercaptophénylacétique, le dithiothréitol, le benzylmercaptan et les mélanges de ceux-ci.

9. Polypeptide de formule :
(I) X₁-N(CH₂CH₂SH)₂
ou de formule : 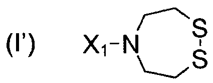
dans lesquelles X₁ représente un fragment peptidique et le groupement -N(CH₂CH₂SH)₂ ou 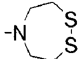 est lié à la terminaison C=O du résidu d'acide aminé du fragment peptidique X₁ qui est en position C-terminale.

10. Procédé de fabrication d'un polypeptide de formule :
(I) X₁-N(CH₂CH₂SH)₂
X₁ représentant un fragment peptidique et le groupement -N(CH₂CH₂SH)₂ étant lié à la terminaison C=O du résidu d'acide aminé du fragment peptidique X₁ qui est en position C-terminale, comprenant au moins une étape de synthèse peptidique et une étape de fonctionnalisation C-terminale.

11. Procédé selon la revendication 10, dans lequel l'étape de synthèse peptidique précède l'étape de fonctionnalisation ; l'étape de synthèse peptidique fournit un polypeptide de formule :
(IX) X₁-OH
comportant de préférence des groupements protecteurs sur ses fonctions amines et carboxyliques, à l'exception de sa fonction carboxylique C-terminale ; et l'étape de fonctionnalisation comprend :
- la réaction du polypeptide de formule (IX) avec le composé amine de formule :
(VIII) NH(CH₂-CH₂-S-G₁)₂
dans laquelle G₁ représente un groupement protecteur, ledit groupement protecteur formant de préférence une fonction thioéther, thioester ou disulfure, et étant de manière plus particulièrement préférée le groupement triphénylméthyle, en phase liquide, pour former le polypeptide de formule (I) ;
- éventuellement la déprotection du polypeptide de formule (I).

12. Support de résine polymère pour la synthèse de polypeptides en phase solide, comprenant un squelette principal et des groupements fonctionnels NH-(CH₂CH₂-S-Trt-)₂ ou des groupements fonctionnels NH-(CH₂CH₂-S-Trt-CO-NH-)₂, ou des groupements fonctionnels G₂-AA-N-(CH₂CH₂-S-Trt-)₂ ou des groupements fonctionnels G₂-AA-N-(CH₂CH₂-S-Trt-CO-NH-)₂, où Trt représente un groupement triphénylméthyle éventuellement substitué par un ou plusieurs substituants, notamment choisis parmi les substituants chlore, méthoxy, méthyle, fluor et cyano ; AA représente un résidu d'acide aminé comportant éventuellement un ou plusieurs groupements protecteurs ; G₂ représente un atome d'hydrogène ou un groupement protecteur de fonction amine ; dans lequel les groupements fonctionnels NH-(CH₂CH₂-S-Trt-)₂ ou G₂-AA-N(CH₂CH₂-S-Trt-)₂ sont liés au squelette principal par les deux groupements triphénylméthyle, ou les groupements fonctionnels NH-(CH₂CH₂-S-Trt-CO-NH-)₂ ou G₂-AA-N-(CH₂CH₂-S-Trt-CO-NH-)₂ sont liés au squelette principal par les deux groupements amines.

13. Support de résine polymère selon la revendication 12, dans lequel le squelette principal est choisi parmi les squelettes polystyrène, polyacrylamide, polyéthylèneglycol, cellulose, polyéthylène, polyester, latex, polyamide, polydiméthylacrylamide, copolymère polyéthylèneglycol-polystyrène, copolymère polyéthylèneglycol-polyacrylamide et leurs dérivés.

14. Procédé selon la revendication 11, dans lequel :
- l'étape de fonctionnalisation précède l'étape de synthèse peptidique ;
- l'étape de fonctionnalisation comprend :
■ le couplage d'un acide aminé à un support de résine polymère selon la revendication 12 comprenant un squelette principal et des groupements fonctionnels NH-(CH₂CH₂-S-Trt-)₂ ou des groupements fonctionnels NH-(CH₂CH₂-S-Trt-CO-NH-)₂, pour fournir un support amorce ; ou
■ la fourniture d'un support amorce qui est un support de résine polymère selon la revendication 12 comprenant un squelette principal et des groupements fonctionnels G₂-AA-N-(CH₂CH₂-S-Trt-)₂ ou des groupements fonctionnels G₂-AA-N-(CH₂CH₂-S-Trt-CO-NH-)₂;
- l'étape de synthèse peptidique comprend une succession de couplages d'acides aminés sur le support amorce.

15. Procédé de fabrication d'un polypeptide de formule : 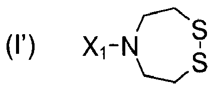
dans laquelle X₁ représente un fragment peptidique et le groupement 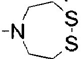 est lié à la terminaison C=O du résidu d'acide aminé du fragment peptidique X₁ qui est en position C-terminale, comprenant une étape d'oxydation d'un polypeptide de formule :
(I) X₁-N(CH₂CH₂SH)₂
de préférence au contact de l'air ,ou en présence de I₂ ou de diamide, et dans un tampon, ladite étape d'oxydation étant de préférence précédée d'une étape de fabrication du polypeptide de formule (I) selon le procédé des revendications 10, 11, ou 14.

## Patentansprüche

1. Verfahren zur Herstellung eines Polypeptids der Formel:
(III) X₁-X"-X₂
wobei X₁ und X₂ jeweils für ein Peptidfragment stehen und X" für einen Aminosäurerest steht, der eine Thiolfunktion aufweist,
wobei das Verfahren mindestens einen Reaktionsschritt der Ligation zwischen einem Polypeptid der Formel:
(I) X₁-N(CH₂CH₂SH)₂
und einem Polypeptid der Formel:
(II) H-X"-X₂
umfasst.

2. Verfahren nach Anspruch 1, wobei die Ligationsreaktion durchgeführt wird, indem ein Polypeptid der Formel: 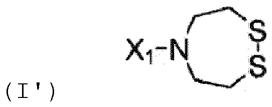
mit dem Polypeptid der Formel (II) in Kontakt gebracht wird, in Gegenwart mindestens einer Verbindung, die Disulfidbindungen reduziert, wobei das Polypeptid der Formel (I') für die Ligationsreaktion *in situ* zum Polypeptid der Formel (I) reduziert wird.

3. Verfahren nach Anspruch 1 oder 2, wobei X₂ für ein Peptidfragment der folgenden Formel steht
(IV) X₂'-(Xᵢ"-X'ᵢ)_{i=3...n}
wobei n eine ganze Zahl ist, die größer oder gleich 3 ist, wobei jedes Xᵢ", bei einem Zahlenwert i von einer ganze Zahl im Bereich von 3 bis n, für einen Aminosäurerest steht, der eine Thiolfunktion aufweist, und wobei jedes Xᵢ' , bei einem Zahlenwert i von einer ganzen Zahl im Bereich von 2 und n, für ein Peptidfragment steht;
wobei das Verfahren, im Vorfeld des Reaktionsschrittes der Ligation zwischen dem Polypeptid der Formel (I) und dem Polypeptid der Formel (II), eine Abfolge von n-2 Ligationsreaktionsschritten umfasst, wobei es sich bei dem Ligationsreaktionsschritt mit der laufenden Nummer j, bei einem Zahlenwert j von einer ganzen Zahl im Bereich von 1 bis n-2, um eine Ligationsreaktion zwischen einem Polypeptid der Formel:
(V) H-Xₙ₋ⱼ"-Xₙ₋ⱼ'- N(CH₂CH₂SH)₂
wobei die Aminfunktion und/oder die Thiolfunktion des Restes Xₙ₋ⱼ" geschützt ist,
und einem Polypeptid der Formel:
(VI) % H-(Xᵢ"-Xᵢ')_{i=(n-j+1)...n}
handelt, welche dazu dient, ein Polypeptid der Formel:
(VII) H-(X₁"-Xᵢ')_{i=(n-1)...n}
zu bilden, wobei bei dem Polypeptid der Formel (VII) nach Abschluss der Ligationsreaktion die Schutzgruppe von der Thiolfunktion des Restes Xₙ₋ⱼ" entfernt wird.

4. Verfahren nach Anspruch 3, wobei ein oder mehrere der n-2 Schritte der Ligationsreaktion zwischen dem Polypeptid der Formel (V) und dem Polypeptid der Formel (VI) durchgeführt wird/werden, indem ein Polypeptid der Formel: 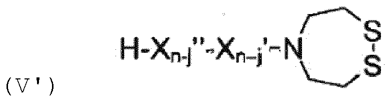 mit dem Polypeptid der Formel:
(VI) H-(Xᵢ"-Xᵢ')_{i=(n-j+1)...n}
in Kontakt gebracht wird, wobei j eine ganze Zahl im Bereich von 1 bis n-2 ist, in Gegenwart mindestens einer Verbindung, die Disulfidbindungen reduziert.

5. Verfahren zur Herstellung eines zyklischen Polypeptids nach der Formel: 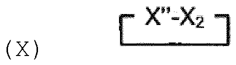
wobei X₂ für ein Peptidfragment steht und X" für einen Aminosäurerest steht, der eine Thiolfunktion aufweist,
wobei das Verfahren mindestens einen Reaktionsschritt der Ligation eines Polypeptids der Formel:
(XI) H-X"-X₂-N (CH₂CH₂SH)₂
mit sich selbst umfasst.

6. Verfahren nach Anspruch 5, wobei die Ligationsreaktion durchgeführt wird, indem ein Polypeptid der Formel: 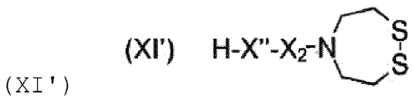
mit mindestens einer Verbindung, die Disulfidbindungen reduziert, in Kontakt gebracht wird, wobei das Polypeptid der Formel (XI') für die Ligationsreaktion *in situ* zum Polypeptid der Formel (XI) reduziert wird.

7. Verfahren nach einem der Ansprüche 1 bis 6, wobei die Ligationsreaktion(en) in wässrigem Milieu durchgeführt wird/werden, vorzugsweise bei einem pH im Bereich von 6,5 bis 8,5, auf besonders bevorzugte Weise von 7 bis 8, und idealerweise von ungefähr 7,5.

8. Verfahren nach einem der Ansprüche 1 bis 7, wobei die Ligationsreaktion(en) in Gegenwart mindestens einer Verbindung durchgeführt wird/werden, die Disulfidbindungen reduziert, wobei diese vorzugsweise aus tris(2-Carboxyethyl)phosphin, 4-Mercaptophenylessigsäure, Dithiothreitol, Benzylmercaptan und deren Mischungen ausgewählt ist.

9. Polypeptid der Formel:
(I) X₁-N(CH₂CH₂SH)₂
oder der Formel: 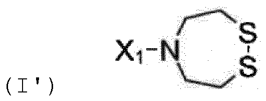
wobei X₁ für ein Peptidfragment steht und die Gruppe - N(CH₂CH₂SH)₂ oder 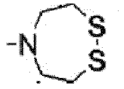 an die endständige Gruppe C=O des Aminosäurerestes der Peptidfragments X₁, welche sich in C-terminaler Stellung befindet, gebunden ist.

10. Verfahren zur Herstellung eines Polypeptids nach der Formel:
(I) Xₗ-N(CH₂CH₂SH)₂
wobei X₁ für ein Peptidfragment steht und die Gruppe - N(CH₂CH₂SH)₂ an die endständige Gruppe C=O des Aminosäurerestes der Peptidfragments X₁, welche sich in C-terminaler Stellung befindet, gebunden ist, wobei das Verfahren mindestens einen Schritt der Peptidsynthese und einen Schritt des Einfügens einer C-terminalen funktionellen Gruppe umfasst.

11. Verfahren nach Anspruch 10, wobei der Peptidsyntheseschritt dem Schritt des Einfügens einer funktionellen Gruppe vorangeht; der Peptidsyntheseschritt ein Polypeptid der folgenden Formel liefert:
(IX) X₁-OH
dessen Amin- und Carbonsäurefunktionen vorzugsweise mit Schutzgruppen versehen sind, mit Ausnahme von dessen C-terminaler Carbonsäurefunktion; und der Schritt des Einfügens einer funktionellen Gruppe Folgendes umfasst:
- Umsetzen des Polypeptids der Formel (IX) mit der Aminverbindung der Formel:
(VIII) NH(CH₂-CH₂-S-G₁)₂
wobei G₁ für eine Schutzgruppe steht, wobei die Schutzgruppe vorzugsweise eine Thioether-, Thioester oder Disulfidgruppe bildet, und wobei es sich mit besonderem Vorzug um die Triphenylmethylgruppe, in Flüssigphase, handelt, um das Polypeptid der Formel (I) zu bilden;
- möglicherweise das Entfernen der Schutzgruppe vom Polypeptid der Formel (I).

12. Träger aus Polymerharz für die Synthese von Polypeptiden in der Festphase, umfassend ein Hauptgerüst sowie funktionelle Gruppen NH-(CH₂CH₂-S-Trt-)₂ oder funktionelle Gruppen NH-(CH₂CH₂-S-Trt-CO-NH-)₂, oder funktionelle Gruppen G₂-AA-N-(CH₂CH₂-S-Trt-)₂ oder funktionelle Gruppen G₂-AA-N-(CH₂CH₂-S-Trt-CO-NH-)₂, wobei Trt für eine Triphenylmethylgruppe steht, die möglicherweise mit einem oder mehreren Substituenten substituiert ist, die insbesondere aus den Substituenten Chlor, Methoxy, Methyl, Fluor und Cyano ausgewählt sind; AA für einen Aminosäurerest steht, der möglicherweise eine oder mehrere Schutzgruppen aufweist; G₂ für ein Wasserstoffatom oder eine Aminofunktions-Schutzgruppe steht; wobei die funktionellen Gruppen NH-(CH₂CH₂-S-Trt-)₂ oder G₂-AA-N(CH₂CH₂-S-Trt-)₂ über die beiden Triphenylmethylgruppen an das Hauptgerüst gebunden sind oder die funktionellen Gruppen NH-(CH₂CH₂-S-Trt-CO-NH-)₂ oder G₂-AA-N-(CH₂CH₂-S-Trt-CO-NH-)₂ über die beiden Amingruppen an das Hauptgerüst gebunden sind.

13. Träger aus Polymerharz nach Anspruch 12, wobei das Hauptgerüst aus den Gerüsten Polystyrol, Polyacrylamid, Polyethylenglykol, Cellulose, Polyethylen, Polyester, Latex, Polyamid, Polydimethylacrylamid, Polyethylenglykol/Polystyrol-Copolymer, Polyethylenglykol/Polyacrylamid-Copolymer und deren Derivaten ausgewählt ist.

14. Verfahren nach dem Anspruch 11, wobei:
- der Schritt des Einfügens einer funktionellen Gruppe dem Schritt der Peptidsynthese vorangeht;
- der Schritt des Einfügens einer funktionellen Gruppe Folgendes umfasst:
• Kuppeln einer Aminosäure an einen Träger aus Polymerharz nach Anspruch 12, welcher ein Hauptgerüst und funktionelle Gruppen NH-(CH₂CH₂-S-Trt-)₂ oder funktionelle Gruppen NH-(CH₂CH₂-S-Trt-CO-NH-)₂ umfasst, um ein geträgertes Anfangsstück zu erhalten; oder
• Bereitstellen eines geträgerten Anfangsstücks, wobei es sich um einen Träger aus Polymerharz nach Anspruch 12 handelt, welcher ein Hauptgerüst und funktionelle Gruppen G₂-AA-N-(CH₂CH₂-S-Trt-)₂ oder funktionelle Gruppen G₂-AA-N-(CH₂CH₂-S-Trt-CO-NH-)₂ umfasst;
- der Schritt der Peptidsynthese eine Abfolge von Kupplungen von Aminosäuren an das geträgerte Anfangsstück umfasst.

15. Verfahren zur Herstellung eines Polypeptids nach der Formel: 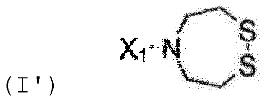
wobei X₁ für ein Peptidfragment steht und die Gruppe 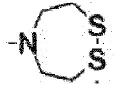 an die endständige Gruppe C=O des Aminosäurerestes der Peptidfragments X₁, welche sich in C-terminaler Stellung befindet, gebunden ist, wobei das Verfahren einen Schritt der Oxidation eines Polypeptids der Formel:
(I) X₁-N(CH₂CH₂SH)₂
umfasst, vorzugsweise in Luftkontakt, oder in Gegenwart von I₂ oder Diamid, sowie in einem Puffer, wobei dem Oxidationsschritt vorzugsweise ein Schritt der Herstellung des Polypeptids der Formel (I) gemäß dem Verfahren der Ansprüche 10, 11 oder 14 vorangeht.

## Claims

1. Method for manufacturing a polypeptide of formula:
(III) X₁-X"-X₂
X₁ and X₂ each representing a peptide fragment, and X" representing an amino acid residue comprising a thiol function, said method comprising at least one step of ligation reaction between a polypeptide of formula:
(I) X₁-N(CH₂CH₂SH)₂
and a polypeptide of formula:
(II) H-X"-X₂.

2. Method according to claim 1, in which the ligation reaction is carried out by bringing a polypeptide of formula: 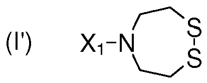
into contact with the polypeptide of formula (II), in the presence of at least one compound that reduces the disulphide bonds, the polypeptide of formula (I') being reduced *in situ* to the polypeptide of formula (I) for the ligation reaction.

3. Method according to claim 1 or 2, in which X₂ represents a peptide fragment of formula
(IV) X₂'-(Xi"-Xi')i_{=3...n}
n being an integer greater than or equal to 3, each Xᵢ", for i an integer comprised between 3 and n, representing an amino acid residue bearing a thiol function, and each Xᵢ', for i an integer comprised between 2 and n, representing a peptide fragment; said method comprising, before the step of ligation reaction between the polypeptide of formula (I) and the polypeptide of formula (II), a succession of n-2 steps of ligation reaction, the j^{th} step of ligation reaction, for j an integer comprised between 1 and n-2, being a ligation reaction between a polypeptide of formula:
(V) H-Xₙ₋j"-Xₙ₋ⱼ'- N(CH₂CH₂SH)₂
in which the amine function and/or the thiol function of the residue Xₙ₋ⱼ" is protected
and a polypeptide of formula:
(VI) H-(Xᵢ"-Xᵢ')_{i=(n-j+1)...n}
to form a polypeptide of formula:
(VII) H-(Xᵢ"-Xᵢ')ᵢ₌₍ₙ₋ⱼ)...ₙ
the polypeptide of formula (VII) undergoing deprotection of the thiol function of the residue Xₙ₋ⱼ" at the end of the ligation reaction.

4. Method according to claim 3, in which one or more of the n-2 steps of ligation reaction between the polypeptide of formula (V) and the polypeptide of formula (VI) is carried out by bringing a polypeptide of formula: 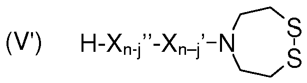 into contact with the polypeptide of formula:
(VI) H-Xᵢ"-Xᵢ')_{i=(n-j+1)...n}
j being an integer comprised between 1 and n-2, in the presence of at least one compound that reduces the disulphide bonds.

5. Method for manufacturing a cyclic polypeptide of formula: 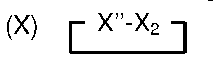
X₂ representing a peptide fragment, and X" representing an amino acid residue comprising a thiol function,
said method comprising at least one step of ligation reaction of a polypeptide of formula:
(Xl) H-X"-X₂-N(CH₂CH₂SH)₂
with itself.

6. Method according to claim 5, in which the ligation reaction is carried out by bringing a polypeptide of formula: 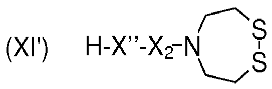
into contact with at least one compound that reduces the disulphide bonds, the polypeptide of formula (XI') being reduced *in situ* to the polypeptide of formula (XI) for the ligation reaction.

7. Method according to one of claims 1 to 6, in which the ligation reaction or ligation reactions are carried out in an aqueous medium, preferably at a pH comprised between 6.5 and 8.5, more particularly preferably between 7 and 8, and ideally of approximately 7.5.

8. Method according to one of claims 1 to 7, in which the ligation reaction or ligation reactions are carried out in the presence of at least one compound that reduces the disulphide bonds, preferably selected from tris(2-carboxyethyl)phosphine, 4-mercaptophenylacetic acid, dithiothreitol, benzyl mercaptan and mixtures thereof.

9. Polypeptide of formula:
(I) X₁-N(CH₂CH₂SH)₂
or of formula: 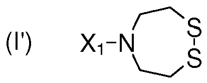
in which X₁ represents a peptide fragment and the group - N(CH₂CH₂SH)₂ or 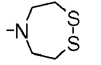
is bound to the C=O termination of the amino acid residue of peptide fragment X₁ that is in C-terminal position.

10. Method for manufacturing a polypeptide of formula:
(I) X₁-N(CH₂CH₂SH)₂
X₁ representing a peptide fragment and the -N(CH₂CH₂SH)₂ group being bound to the C=O termination of the amino acid residue of peptide fragment X₁ that is in C-terminal position, comprising at least one step of peptide synthesis and one step of C-terminal functionalization.

11. Method according to claim 10, in which the step of peptide synthesis precedes the step of functionalization; the step of peptide synthesis supplies a polypeptide of formula:
(IX) X₁-OH
preferably comprising protective groups on its amine and carboxylic functions, with the exception of its C-terminal carboxylic function; and the step of functionalization comprises:
- reaction of the polypeptide of formula (IX) with the amine compound of formula:
(VIII) NH(CH₂-CH₂-S-G₁)₂
in which G₁ represents a protective group, said protective group preferably forming a thioether, thioester or disulphide function, and more particularly preferably being the triphenylmethyl group, in the liquid phase, to form the polypeptide of formula (I);
- optionally deprotection of the polypeptide of formula (I).

12. Polymer resin support for solid-phase synthesis of polypeptides, comprising a main skeleton and NH-(CH₂CH₂-S-Trt-)₂ functional groups or NH-(CH₂CH₂-S-Trt-CO-NH-)₂ functional groups or G₂-AA-N-(CH₂CH₂-S-Trt-)₂ functional groups or G₂-AA-N-(CH₂CH₂-S-Trt-CO-NH-)₂ functional groups, where Trt represents a triphenylmethyl group optionally substituted with one or more substituents, in particular selected from the substituents chlorine, methoxy, methyl, fluorine and cyano; AA represents an amino acid residue optionally bearing one or more protective groups; G₂ represents a hydrogen atom or a group protecting the amine function; in which the NH-(CH₂CH₂-S-Trt-)₂ functional groups or the G₂-AA-N(CH₂CH₂-S-Trt-)₂ functional groups are bound to the main skeleton by the two triphenylmethyl groups, or the NH-(CH₂CH₂-S-Trt-CO-NH-)₂ functional groups or the G₂-AA-N-(CH₂CH₂-S-Trt-CO-NH-)₂ functional groups are bound to the main skeleton by the two amine groups.

13. Polymer resin support according to claim 12, in which the main skeleton is selected from the polystyrene, polyacrylamide, polyethylene glycol, cellulose, polyethylene, polyester, latex, polyamide, polydimethylacrylamide, polyethylene glycol-polystyrene copolymer, polyethylene glycol-polyacrylamide copolymer skeletons and derivatives thereof.

14. Method according to claim 11, in which:
- the step of functionalization precedes the step of peptide synthesis;
- the step of functionalization comprises:
■ coupling an amino acid to a polymer resin support according to claim 12 comprising a main skeleton and NH-(CH₂CH₂-S-Trt-)₂ functional groups or NH-(CH₂CH₂-S-Trt-CO-NH-)₂ functional groups, to supply a primer support; or
■ supplying a primer support which is a polymer resin support according to claim 12 comprising a main skeleton and G₂-AA-N-(CH₂CH₂-S-Trt-)₂ functional groups or G₂-AA-N-(CH₂CH₂-S-Trt-CO-NH-)₂ functional groups;
- the step of peptide synthesis comprises a succession of couplings of amino acids on the primer support.

15. Method for manufacturing a polypeptide of formula: 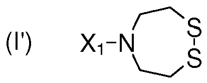
in which X₁ represents a peptide fragment and the 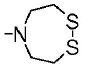 group is bound to the C=O termination of the amino acid residue of peptide fragment X₁ that is in C-terminal position, comprising a step of oxidation of a polypeptide of formula:
(I) X₁-N(CH₂CH₂SH)₂
preferably in contact with the air, or in the presence of I₂ or of diamide, and in a buffer, said oxidation step preferably being preceded by a step of manufacture of the polypeptide of formula (I) according to the method of claims 10, 11, or 14.
